# EUROPÄISCHE PATENTANMELDUNG

(11) **EP 1 333 090 A1**
(43) Veröffentlichungstag der Anmeldung: **06.08.2003**
(21) Anmeldenummer: 02002464.2
(22) Anmeldetag: 01.02.2002
(51) Int. Cl.: C12N 15/11, C12N 15/63, C12N 5/10, A01K 67/00, A61K 31/70, A61K 48/00, C12Q 1/68, A61K 35/00, G01N 33/50

(54) **Prionprotein-spezifische Aptamere**

(71) Anmelder: NascaCell GmbH, 82327 Tutzing (DE)
(72) Erfinder: Proske, Daniela, 81543 München (DE)
(74) Vertreter: Bohmann, Armin K., Dr.

(57) **Zusammenfassung**

Die vorliegende Erfindung betrifft Nukleinsäuren, die an ein Peptid oder Protein binden, das die Aminosäuren 90 bis 129 des Prionproteins umfasst sowie diese Nukleinsäuren umfassende Medikamente, die insbesondere zur Behandlung neurodegenerativer Erkrankungen geeignet sind.

## Beschreibung

Die vorliegende Erfindung betrifft Nukleinsäuren, die in der Lage sind, an ein Protein oder ein Peptid zu binden, das einen Teil des Prionproteins umfasst, die Konversion von PrPc zu PrPSc, die Akkumulation von PrPSc- Molekülen, die Übertragbarkeit (Transmissiblität) von Prionerkrankungen, Prävention von Prionenerkrankungen, die Amyloidausbildung- und akkumulation und die Vermehrung und/oder die Replikation des Erregers bestimmter Erkrankungen zu beeinflussen, diese umfassende Vektoren, Zellen und Tiere, deren Verwendungen, diese umfassende Zusammensetzungen, diese verwendende Screening-Verfahren sowie Verfahren zur Identifizierung und Isolierung von Nukleinsäuren, die in der Lage sind, an ein Zielmolekül zu binden.

### Stand der Technik

### Prionenerkrankungen

Zu den übertragbaren spongiformen Enzephalopathien (TSE) oder auch Prionenerkrankungen gehören unter anderem die Creutzfeld-Jakob-Krankheit (CJD), die Kuru-Krankheit des Menschen, die bovine spongiforme Enzephalopathie des Rindes (BSE) und Scrapie des Schafes. Schon in den sechziger Jahren stellten Alper und Mitarbeiter fest, daß die Scrapie-Proben aus Schafgehirnen gegen Nukleasen und UV-Strahlung resistent sind [Alper et al., Biochem Biophys Res Commun 22 (1966), 278-84]. Im Gegensatz dazu führte die Behandlung mit Protein-denaturierenden Agentien, wie z.B. 8 M Harnstoff oder Phenol zum Verlust der Infektiösität [Alper et al., Nature 214 (1967) 764-6]. Griffith postulierte bereits in Jahr 1967, daß der Erreger dieser neurodegenerativen Erkrankungen ein infektiöses Eiweißpartikel sein könnte, welches sich ohne die Beteiligung einer Nukleinsäure vermehrt [Griffith, Nature 215 (1967) 1043-4]. Fast 15 Jahre später gelang es Prusiner und seinen Mitarbeitern ein Protein aus Gehirnen infizierter Hamster zu isolieren, welches den von Griffith beschriebenen Eigenschaften entsprach. Prusiner führte dafür den Begriff Prionprotein (PrP) ein, die Kurzform für ein proteinartiges infektiöses Partikel (engl. proteinaceous infectious particle) [Prusiner et al., Science 216 (1982) 136-44]. Die Charakterisierung des Scrapie Erregers ergab, daß zu dem pathogenen Protein ein zelluläres Homolog existiert, wobei die infektiöse PrP-lsoform (PrP^{Sc}, ^{Sc} = Scrapie) durch einen postranslationalen, bisher noch wenig verstandenen Prozess, aus der zellulären Isoform (PrP^{C}) entsteht [Oesch et al., Cell 40 (1985) 735-46; Horwich et al., Cell 89 (1997) 499-510].

### Prionenerkrankungen des Menschen

Spongiforme Enzephalopathien (Prionenerkrankungen) des Menschen haben sich als durch eine Infektion erworbene aber auch vererbare und sporadisch auftretende Erkrankungen manifestiert. Eine Übersicht der Prionenerkrankungen findet sich in Tabelle 1.

**Tabelle 1**

| *Neurodegenerative Erkrankungen, insbesondere spongioforme Encephalopathien, wie Prionenerkrankungen des Menschen* | |
|---|---|
| *Erkrankung* | *Ätiologie* |
| Kuru | Infektion |

| Creutzfeldt-Jakob-Krankheit (CJD) | |
|---|---|
| -latrogen (iCJD) | Infektion |
| -Sporadisch (sCJD) | unbekannt |
| -Infektiös (nvCJD) | Infektion |
| -Familiär (fCJD) | PrP-Mutation |
| Gerstmann Sträussler Scheinker Syndrom (GSS) | PrP-Mutation |
| fatale familiäre Insomie (FFI) | PrP-Mutation |

Die CJD-Krankheit tritt in den meisten Fällen sporadisch (sCJD) auf (80-90%), ohne daß eine Infektionsquelle oder eine genetische Prädisposition feststellbar sind. Die sCJD betrifft in der Regel Patienten im sechsten bis siebten Lebensjahrzehnt. Erste Auffälligkeiten sind progressive Demenz und verschiedene weitere neurologische Symptome, wie Ataxie und Myoklonien [Kretzschmar, MMW 89 (2000) 34-38]. Wenige Fälle von CJD (iCJD) werden auf iatrogene Infektionen bei medizinischen Behandlungen zurückgeführt, beispielsweise bei Hornhautverpflanzungen am Auge, durch Verwendung kontaminierter chirugischer Instrumente oder Injektion von Wachstumshormonen aus Hypophysen von CJD-erkrankten Patienten [Masson et al., Neurology 44 (1994) 179-80]. Neben den iatrogenen und sporadischen Fällen treten in etwa 10% heriditäre Formen auf, wie GSS, FFI und fCJD. Heriditäre Prionkrankheiten lassen sich relativ einfach durch Untersuchung der Sequenz des Prionproteingens erkennen. Bei einer in Deutschland durchgeführten CJD-Untersuchung wurden von 1993 bis 1998 insgesamt 587 Verdachtsfälle molekulargenetisch analysiert. Dabei war bei 40 Patienten eine pathogene Mutation am Prionprotein nachweisbar [Prusiner, Brain Pathol 8 (1998) 499-513, Kretzschmar, MMW 89 (2000), 34-38]. Das pathoanatomische Korrelat der CJD-Krankheit ist charakterisiert durch eine schwammartige Zersetzung des Gehirns (Spongiosis) bzw. Vakuolisierung in Folge von Ablagerungen des Prionproteins (PrP^{Sc}) und dem Absterben neuronaler Zellen. Sie geht mit Störungen verschiedener Körperfunktionen einher, wie Gedächtnisverlust, Sehschwierigkeiten und motorischen Störungen. Kuru oder "der lachende Tod" wurde erstmals bei einem Stamm im Hochland von Papua-Neuguinea von V. Zigras und C. Gajdusek beobachtet. Kuru soll durch rituellen Kanibalismus übertragen worden sein und ist charakterisiert durch den Verlust der Bewegungskoordination (Ataxie), später auch der geistigen Fähigkeiten [Gajdusek, Curr Top Microbiol Immunol 40 (1967), 59-63]. Nach dem Auftreten der ersten Symptome führen Prionenerkrankungen typischerweise über progressive Demenz zum Tod [Prusiner, Curr Top Microbiol Immunol 207 (1996), 1-17].

### BSE - Übertragbarkeit auf den Menschen ?

Der epidemieartige Ausbruch der Rinderseuche BSE im Jahr 1986 in Großbritanien hatte mit 3500 Neuerkrankungen pro Monat in den Jahren 1992 und 1993 seinen Höhepunkt erreicht [Anderson et al., Nature 382 (1996), 779-88]. Ursache für den Anstieg war, so vermutet man, die Verfütterung von Tiermehl, welches aus Scrapie enthaltenen Schafskadavern und BSE enthaltenen Rindskadavern gewonnen wurde [Wilesmith et al., Vet Rec 128 (1991), 199-203]. Eine Übertragung auf den Menschen wurde zu diesem Zeitpunkt von der britischen Regierung ausgeschlossen und ca. 750000 infizierte Rinder gelangten in die menschliche Nahrungskette.

Im März 1996 berichtet eine englische Forschergruppe von einer neuen Variante der Creutzfeld-Jakob-Krankheit (nvCJD) [Will et al., Lancet 347 (1996), 921-5]. Diese hat mit großer Wahrscheinlichkeit ihren Ursprung in der bovinen spongiformen Enzephalopathie. Dabei unterscheidet sich die neue CJD-Variante pathologisch von den bisher beobachteten CJD-Fällen durch das Auftreten von vermehrten PrP^{Sc}-Ablagerungen in Form von floriden (blütenförmigen) Plaques im Gehirn, welche denen von Kuru Patienten und BSE-infizierten Halbaffen sehr ähnlich sind [Lasmezas et al., J Gen Virol 77 (1996), 1601-9]. Weiterhin sind die an nvCJD verstorbenen Patienten mit einem Durchschnittsalter von 29 Jahren auffällig jung und die Krankheitsdauer, d.h. das Auftreten der ersten Symptome bis zum Tod, ist mit mehr als sechs Monaten länger als bei den bisher beschriebenen spongiformen Enzephalopathien des Menschen [Kretzschmar, MMW 89 (2000), 34-38]. Erste Hinweise auf einen Zusammenhang von nvCJD und BSE wurden 1997 von den Arbeitsgruppen um M. Bruce und J. Collinge erbracht. Bruce konnte zeigen, daß sowohl die Pathologie als auch die Inkubationsdauer der mit BSE-und nvCJD-Hirnmaterial infizierten Mäuse im Gegensatz zu den Scrapie-infizierten Mäusen fast identisch sind. Collinge untersuchte das Glykosilierungsmuster von Proben aus nvCJD und BSE Gehirnen im Vergleich zu den sCJD und den iCJD Proben nach elektrophoretischer Auftrennung [Bruce et al., Nature 389 (1997), 498-501], [Hill et al., Nature 389 (1997),448-50, 526]. Er konnte feststellen, daß die nvCJD und BSE-Proben im Gegensatz zu dem Hirnmaterial aus den Patienten, welche an sCJD oder iCJD verstorben sind, ein gemeinsames Glykosilierungsmuster aufweisen. Allerdings sind die von Collinge beschriebenen Unterscheidungskriterien sehr umstritten [Parchi et al., Nature 386 (1997), 232-4]. Weiterhin wurde die potentielle Übertragung von BSE auf den Menschen an transgenen Tiermodellen untersucht.

### Transgene Tiermodelle und Speziesbarriere

Die experimentelle Übertragung von spongiformer Enzephalopathie innerhalb einer Art gelingt effizienter als eine Übertragung zwischen unterschiedlichen Spezies. Man spricht bei diesem Phänomen von der "Speziesbarriere". Beispielsweise entwickeln Mäuse nach Inokulation mit infizierten Hamsterhirnmaterial keine typischen Symptome innerhalb ihrer durchschnittlichen Lebensdauer von 600 Tagen. Man vermutet als Ursache für diese "Speziesbariere" die unterschiedlichen Primärstrukturen der Prionproteine von Donor und Empfänger [Prusiner et al., Cell 63 (1990), 673-86].

Durch Einführung eines PrP-Transgenes in eine Wildtyp-Maus wird die sogenannte Speziesbarriere im Hinblick auf eine Erkrankung nach Erstinfektion durchbrochen. Eine wesentliche Verstärkung dieses Effektes tritt auf, wenn das endogene PrP-Gen der Maus ausgeschaltet wird [Telling et al., Cell 83 (1995), 79-90]. Mit der Generierung von transgenen Mäusen, bei welchen das endogene Maus PrP durch das humane PrP oder bovine PrP ersetzt wurde, sollte ein Modell geschaffen werden, auf dessen Basis der Zusammenhang von nvCJD und BSE im Vergleich untersucht werden kann. Dabei zeigte sich für die transgene "bovine PrP" Maus nach Infektion mit BSE oder nvCJD ein einheitliches Bild hinsichtlich der Pathologie der klinischen Symtomatik insbesondere für die Inkubationszeiten, von dem sich eine mit sCJD infizierte transgene Maus deutlich unterschied [Scott et al., Proc Natl Acad Sci U S A 96 (1999), 15137-42]. Umgekehrt ist die experimentelle Übertragung von nvCJD-Prionen und BSE-Prionen auf transgene Mäuse, welche humanes PrP exprimieren, nur sehr ineffizient bzw. mit einer langen Inkubationsdauer verbunden, während eine Infektion mit sCJD oder iCJD-Material durch eine typische kürzere Inkubationsdauer von ca. 260 Tagen charakterisiert ist [Hill et al., Nature 389 (1997), 448-50, 526].

Hill et al. [Hill et al., Proc Natl Acad Sci U S A 97 (2000), 10248-53] konnten kürzlich zeigen, daß Mäuse, welche mit Hamsterprionen infiziert wurden, zunächst zwar keine typischen klinischen Symptome entwickeln, jedoch nach 600 Tagen und länger Träger des Erregers sind. Inokuliert man die Gehirnhomogenate dieser Mäuse in einer zweiten experimentellen Passage in Hamster und Mäuse so zeigen beide Tierarten innerhalb der typischen Inkubationszeit Krankheitssymptome. Damit stellte er in seinen Studien die "Speziesbarriere" und deren Definition in Frage.

Mit dem Auftreten von spektakulären CJD Erkrankungsfällen bei Kindern im Alter von 12 bis 14 Jahren, der Übertragung von Scrapie auf Schafe durch Injektion von infektiösem Blut und der potentiellen intrauterinen Übertragbarkeit von CJD von der Mutter auf das Kind sind die Prionerkrankungen auch ins Zentrum der öffentlichen Diskussion bei Medien und politischen Entscheidungsgremien gerückt [Bonn, Lancet 356 (2000), 570; Andrews et al., Lancet 356 (2000), 481-2]. Eine routinemäßige Anwendung eines sensitiven BSE-Tests bei allen geschlachteten Tieren, die älter als 30 Monate sind, ist zum gegenwärtigen Zeitpunkt bereits gesetzlich vorgeschrieben.

### Das Prion-Protein (PrP)

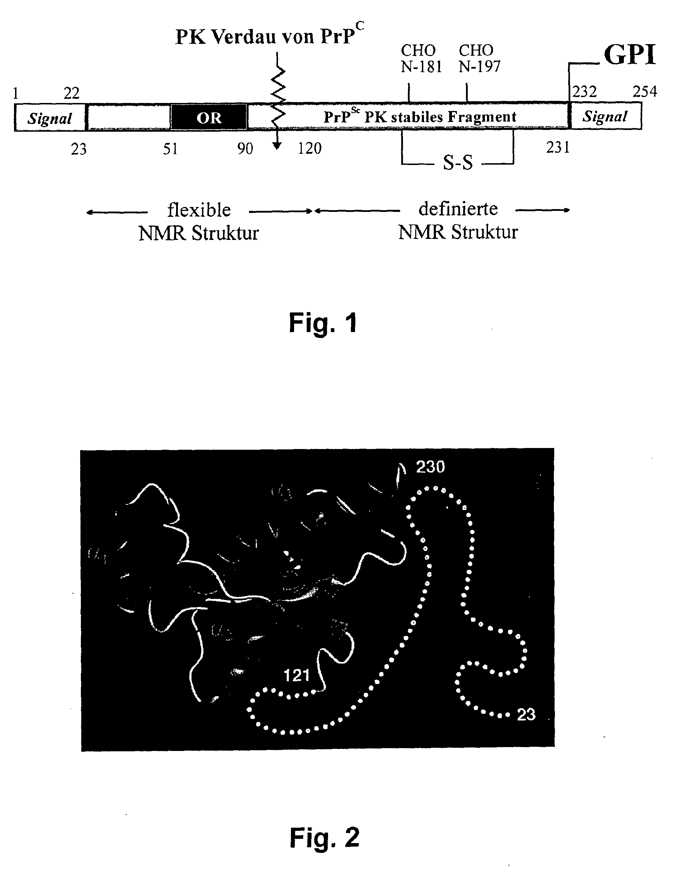

Das zelluläre Prionprotein (PrP^{C}) wird in allen bisher untersuchten Säugertierarten und einigen Vogelarten vor allem im Gehirn exprimiert [Wopfner et al., J Mol Biol 289 (1999), 1163-78]. Innerhalb der Primaten weist das Protein eine Aminosäueresequenzhomologie von 92,9 - 99,6% auf, womit es zur Klasse der hochkonservierten Proteine zählt [Schatzl et al., J Mol Biol 245 (1995), 363-74]. Die PrP m-RNA wird vollständig von einem Exon des singulären Prnp-Gens kodiert, welches beim Menschen auf dem Chromosom 20 lokalisiert ist. Die Primärstruktur des Prionproteins, welches aus gesunden (PrP^{C}) und Scrapie infizierten Tieren (PrP^{Sc}) isoliert wurde, ist identisch [Basler et al., Cell 46 (1986), 417-28] (vgl. Fig. 1). Das Prionprotein existiert in zwei Isoformen, der zellulären Isoform PrP^{C} und der pathogenen Isoform PrP^{Sc}, welche sich trotz identischer Primärstruktur biochemisch und biophysikalisch stark unterscheiden, wie in Tabelle 2 dargestellt.

**Tabelle 2:**

| *Unterschiede zwischen PrP*^{*C*} *und PrP*^{*Sc*}*: biochemische, physikalische und pathologische Aspekte, * zweifach glykosilierte PrP-Formen* | | |
|---|---|---|
| | ***Zellulär (PrP***^{***C***}***)*** | ***Pathogen (PrP***^{***Sc***}***)*** |
| PK-Resistenz | sensitiv | Patielle Resistenz |
| Löslichkeit in Detergentien | löslich | unlöslich |
| Struktur | α-Helix und Loops | β-Faltblatt |
| Molekulargewicht (-pK)* | 33-35kDa | 33-35kDa |
| Molekulargewicht (+pK)* | degradiert | 27-30kDa |
| Halbwertzeit | Kurz (2-4 h) | Lang (> 24 h) |
| Infektiosität | nicht infektiös | infektiös |
| Lokalisierung | Zelloberfläche, verankert | GPI- Sekundäre Lysosomen, [McKinley et al., Lab Invest 65 (1991), 622-30] Ablagerungen |

Man geht davon aus, daß PrP^{C} durch einen posttranslationalen Prozess in PrP^{Sc} konvertiert werden kann, wobei sowohl der Mechanismus der Konversion als auch die Beteiligung zusätzlicher Faktoren noch immer unbekannt ist. Durch CD-Messungen und Fourier-Transform-Infrarotspektroskopie (FTIR) konnte eine deutliche Verschiebung des α-helikalen Anteils von PrP^{C} (42% α-helikal, 3% β-Faltblatt) in eine von β-Faltblättern dominierende Sekundärstruktur von PrP^{Sc} (30% α-helikal, 45% β-Faltblatt) festgestellt werden [Pan et al., Proc Natl Acad Sci U S A 90 (1993), 10962-6]. Mit der Veränderung der Sekundärstruktur und der Tertiärstruktur verändert sich sowohl das Löslichkeitsverhalten von PrP^{Sc} in nichtionischen Detergentien, als auch die Resistenz gegenüber einer Behandlung mit Proteinase K. Außerdem weist PrP^{Sc} eine hohe Tendenz zur Aggregatbildung auf. Pathologisch führt dieser Prozess zu fibrillären Ablagerungen im Gehirn und makroskopisch zu einer schwammartigen Durchlöcherung des Gewebes [Prusiner, Brain Pathol 8 (1998), 499-513]. Infektiösität konnte bisher nur für die PrP^{Sc}-Isoform nachgewiesen werden. Verschiedene Versuche *in vitro* Infektiösität für rekombinantes PrP zu erzeugen und diese im Tierversuch nachzuweisen, waren bisher erfolglos [Post et al., Biol Chem (1998), 379 (11), 1307-17].

### Primär-und Tertiästruktur des Prionproteins

In Fig. 1 ist schematisch der Aufbau der 254 Aminosäure langen Primärstruktur des Hamster-PrP dargestellt. Das Signalpeptid (Aminosäure 1-22) sowie die Signalsequenz (Aminosäure 232-254) am C-Terminus werden durch posttranslationale Modifikation abgespalten [Basler et al., Cell 46 (1986), 417-28; Oesch et al., Cell 40 (1985), 735-46]. Durch einen Glykosylphosphatidylinosit-(GPI)-Anker ist das Protein über den Serinrest an Position 231 mit der Zellmembran verankert [Stahl et al., Cell 51 (1987), 229-40]. Während das zelluläre PrP durch Proteinase K Behandlung vollständig degradiert wird, führt ein Proteinase K-Verdau der infektiösen Isoform lediglich zur Abspaltung des N-Terminus (AS 23-90). Diese verkürzte PrP^{Sc}-Form, auch als PrP^{res} bezeichnet [Caughey et al., J Biol Chem 266 (1991), 18217-23, wird aufgrund ihres Laufverhaltens im SDS-Gel auch als 27-30 KDa-PrP bezeichnet [Bolton et al., Science 218 (1982), 1309-11]. Sowohl PrP^{Sc} als auch PrP^{C} können als di-, mono- und nicht glykosilierte Form nachgewiesen werden. Dabei stellen die Asparaginreste N-181 und N-197 die Glykosilierungspositionen dar [Bolton et al., J Virol 53 (1985), 596-606]. In der Region AS 51-90 befinden sich fünf hintereinanderliegende Glycin- und Prolinreiche Octapeptidsequenzen (sog. Octarepeats). Zusätzliche Octarepeatinsertionen im Prionprotein werden mit verschiedenen klinischen Formen von vererbaren spongiformen Enzephalopathien des Menschen in Zusammenhang gebracht. Während 4 bis 7 Octarepeatinsertionen zu Prionproteinablagerungen im Gehirn führen, werden bei 8 bis 9 Octarepeatinsertionen Plauquebildungen beobachtet wurden [Vital et al., Neuropathol Appl Neurobiol 24 (1998), 125-30].

Im oxidierenden Millieu wird die Struktur des Prionproteins durch die Ausbildung einer Disulfidbrücke zwischen Cystein 179 und Cystein 214 stabilisiert. Diese Tatsache nutzte die Gruppe von Glockshuber und Wüthrich experimentell aus, um erstmals große Mengen an murinem PrP periplasmatisch in *E.coli* zu exprimieren. Damit gelang die Aufklärung der ersten dreidimensionalen Struktur einer N-terminal verkürzten Domäne des Maus-PrP (AS 121-231) durch NMR-Spektroskopie [Riek et al., FEBS Lett 413 (1996), 282-8].

Bis heute wurde von folgenden Spezies die NMR-Struktur des Prionproteins aufgeklärt: das murine PrP [Riek et al., FEBS Lett 413 (1997), 282-8], das PrP des Syrischen Goldhamsters [Donne et al., Proc Natl Acad Sci U S A 94 (1997), 13452-7], das humane PrP [Zahn et al., Proc Nal Acad U S A 97 (2000), 145-50] und im Mai 2000 das bovine PrP [Garcia et al., PNAS 97 (2000), 8334-8339].

Die NMR-Struktur des humanen Prionproteins der vollen Länge (AS23-230) besteht aus einer strukturierten, globulären Domäne (AS125-228) und der in Fig. 2 dargestellten flexiblen N-terminalen Domäne. Aufgrund der Unlöslichkeit von PrP^{Sc} liegen dafür weder NMR-Strukturuntersuchungen noch Röntgenkristallstrukturdaten vor.

### Die physiologische Funktion des Prionproteins

Die Konstruktion von knock-out Mauslinien ist eine etablierte Methode, um die biologische Funktion eines Proteins aufzuklären [Brandon et al., Curr Biol 5 (1995), 873-81]. Die Herstellung von PrPknock-out (PrP^{0/0})-Mäusen führte in drei unterschiedliche Arbeitsgruppen zu unterschiedlichen Phänotypen. Die im Weissmann Labor erzeugte PrP^{0/0}-Maus (ZrCh/C57Bl-129/Sv), bei welcher 80% des PrP-Gens durch Insertion einer Neomycin-Kassette ersetzt wurde, entwickelt sich vollkommen normal und zeigt keine Verhaltensauffälligkeiten oder neurologische Fehlfunktionen [Bueler et al., Nature 356 (1992), 577-82]. Auch die in Edinburgh erzeugte PrP^{0/0}-Maus (RML/129-Ola) zeigt keine phänotypischen Veränderungen. Tobler und Mitarbeiter beobachteten später bei diesen beiden PrPdefizienten Mauslinien (ZrCh und RML) Veränderungen im zirkadianen Rhythmus im Vergleich zur Wildtyp-Maus [Tobler et al., Nature 380 (1996), 639-42]. Eine dritte von einer japanischen Gruppe generierte PrP-knock-out-Mauslinie (*Ngsk Prnp PrP*^{*0*}^{/}^{*0*} / C57Bl-129/Sv) ist charakterisiert durch den Verlust von Purkinje-Neuronen und den damit verbunden Ataxiesymptomen [Sakaguchi et al., Nature 380 (1996), 528-31]. Es wird diskutiert, daß letzterer phänotypische Unterschied zur "Weissmann"-Maus auf eine zusätzliche Deletion eines Purkinje-Zell-spezifischen Enhancers in der 3' nichtkodierenden Region (3'UTR) des PrP-Gens zurückzuführen ist. Allen Knock-out-Mäusen gemeinsam ist die Resistenz gegenüber Infektion durch Scrapie-Material [Bueler et al., Cell 73 (1993), 1339-47; Prusiner et al., Proc Natl Acad Sci U S A 90 (1993), 10608-12].

Die Scrapie-Suszeptibilität der PrP defizienten Mäuse kann durch Expression des Wildtyp-PrP's oder PrP-Varianten, die N-terminal um 49 Aminosäuren verkürzt sind, wieder hergestellt werden [Fischer et al., Embo J 15 (1996), 1255-64].

Weiter wurde mit transgenen Mäusen, die anstelle des endogenen PrP^{C} mit PrP-Konstrukten unterschiedlicher N-terminaler Verkürzung transfiziert wurden, versucht die physiologische Bedeutung des Prionproteins und seiner einzelnen Domänen näher zu untersuchen [Shmerling et al., Cell 93 (1998), 203-14]. Für die Konstrukte mit Deletionen im Bereich AS 32-106 konnte keine Veränderung des Phänotyps dieser Mäuse dokumentiert werden. Konstrukte mit Deletionen weiterer N-terminaler Aminosäurereste (AS 32-121 und AS 32-134) hingegen erzeugen deutliche Effekte in den Tieren, wie schwere Verhaltensstörungen, Untergewicht, sowie neuronale Veränderungen im Gehirn.

Da diese Phänotypen nicht in PrP^{0/0}-Mäusen auftreten, postulierten die Autoren einen zellulären Faktor π, der kompetitiv an den noch nicht eindeutig identifizierten Prionprotein-Liganden oder - Rezeptor bindet und eine physiologische Reaktion analog dem PrP hervorrufen kann. Entsprechend dieser Modellvorstellung ist das um 80 bzw. um 92 Aminosäuren N-terminal verkürzte PrP-Konstrukt zwar in der Lage den Faktor π zu verdrängen, jedoch selbst nicht funktionell [Shmerling et al., Cell 93 (1998), 203-14]. Westaway ist es gelungen ein Protein zu identifizieren, welches sich 16 kb vom PrP-Genlocus entfernt befindet und auf Primärstrukturebene als auch in den biochemischen Eigenschaften dem PrP sehr ähnlich ist. Dieses neue Protein, welches den zellulären Faktor π repräsentieren könnte, wurde als PrP-LP/Dpl (PrP-like Protein/ Doppel) oder als Doppel bezeichnet [Moore et al., J Mol Biol 292 (1999), 797-817].

Für PrP^{C} konnte eine Caveolin abhängige Bindung von PrP^{C} an die Tyrosinkinase Fyn beobachtet werden, die eine mögliche Rolle von PrP in der intrazellulären Signaltransduktion nahelegt [Mouillet-Richard et al., Science 289 (2000), 1925-8]. Eine direkte physiologische Interaktion von PrP und Caveolin konnte jedoch bisher noch nicht gezeigt werden [Keshet et al., J Neurochem 75 (2000), 1889-97].

Die Frage nach der physiologischen Funktion des Prionproteins wird derzeit noch kontrovers diskutiert.

### Die Replikation des Prionproteins

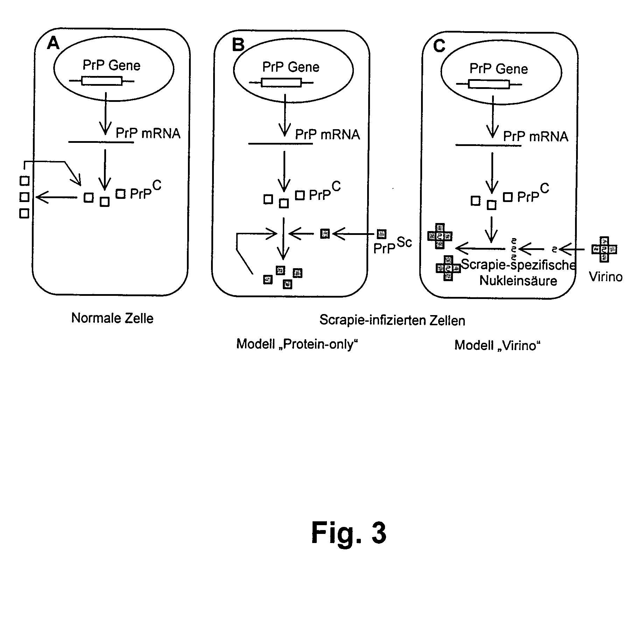

Bis heute existieren zwei sich grundlegend mechanistisch unterscheidende Modelle zur Prionreplikation: das Protein-only-Modell und das Virino-Modell. Mit der Verleihung des Nobelpreises für Medizin an Prusiner (1997), dem Begründer der Protein-only-Hypothese, hat sich dieses Modell in der wissenschaftlichen Gemeinschaft durchgesetzt. Eine schematische Darstellung der Modelle zur Prionpropagation ist in Fig. 3 dargestellt.

### Das Virino-Modell

Während Viren ihre Schutzhülle genetisch selbst bestimmen, soll im Fall der Virino-Hypothese die TSE-spezifische Nukleinsäure zum Schutz vor chemischen oder mechanischen Einflüssen in einen genetisch von der Wirtszelle determinierten Mantel schwer löslichen Amyloides versteckt sein. Dieser Amyloidmantel wird aus PrP^{Sc}-Molekülen gebildet. Das Konzept der virus-induzierten Amyloidose basiert auf verschiedenen Befunden: Es gibt mindestens acht verschiedene TSE-Stämme, die sich in ihren Eigenschaften, wie z. B. der Art und Verteilung der Schädigung und den Inkubationszeiten, unterscheiden [Lasmezas et al., J Gen Virol 77 (1996), 1601-9]. Diese Stämme werden durch Infektion von Versuchstier zu Versuchstier unter Beibehaltung ihrer typischen Charakteristika weitergegeben (passagiert). Dies wurde als Indiz für eine vererbbare Information, somit für die Beteiligung einer genetischen Komponente am Vermehrungsprozess gewertet.

Im Gegensatz dazu begründen die Protein-only-Protagonisten die Stammspezifität mit unterschiedlichen PrP^{Sc}-Konformationen und den damit verbundenen unterschiedlichen Charakteristika [Safar et al., Nat Med 4 (1998), 1157-65].

Die Anhänger der Virinohypothese sehen die Ausbreitung des Erregers nach Infektion über den Magen-Darmtrakt in Analogie zu Viruserkrankungen.

Auf der Suche nach einem infektiösen Partikel ist es Diringer gelungen, im Elektronenmikroskop kleine, symmetrische, den Vorstellungen dieser Theorie ensprechende Strukturen von 10 nm Durchmesser in Scrapie infizierten Hamstergehirnen zu dokumentieren [Ozel et al., Lancet 343 (1994), 894-5]. Ähnliche Partikel identifizierte er später in Gehirnen verstorbener CJD-Pateinten [Ozel et al., Lancet 344 (1994), 923-4]. Eine Infektiösität dieser Strukturen konnte jedoch bisher nicht nachgewiesen werden. Manuelides hat weitere Hinweise für einen virus-ähnlichen und infektiösen Partikel, von ca. 27 nm Größe vorgelegt. [Manuelidis et al., Proc Natl Acad Sci U S A 92 (1995), 5124-8. Aus der selben Arbeitsgruppe stammt eine Publikation die nukleaseresistente RNA-Moleküle von 6000 Basen isoliert aus CJD-Hirnhomogenaten beschreibt [Akowitz et al., Nucleic Acids Res 22 (1994) 1101-7].

Die Gruppe von Riesner jedoch schließt durch verschiedene Studien aus, daß die im infektiösen Partikel enthaltene Nukleinsäure größer als 80 Nukleotide ist [Kellings et al., Arch Virol Suppl 7 (1993), 215-25]. Damit wäre das hypothetische Genom des potentiellen infektiösen Partikels um ein Vielfaches kleiner als das bekannter Viren oder Viroide. Riesner stellt entsprechend seiner Ergebnisse die Existenz eines Scrapie spezifischen Virus in Frage [Kellings et al., Philos Trans R Soc Lond B Biol Sci 343 (1994), 425-30].

### Die Heterodimer Hypothese oder Das Protein-Only-Modell

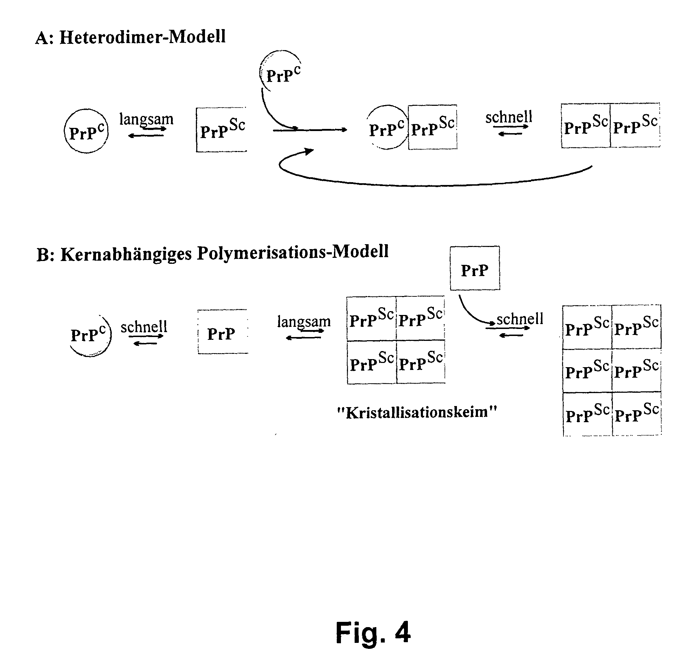

Die Resistenz des infektiösen Agens gegenüber nukleinsäurezerstörenden Behandlungen, die Sensitivität gegenüber proteindenaturierenden Substanzen wie 8 M Harnstoff und Phenol [Alper et al., Biochem Biophys Res Commun 22 (1966), 278-84; Alper et al., Nature 214 (1967), 764-6] sowie die Tatsache, daß keine TSE-spezifische Nukleinsäure, kein Virus oder Virino identifiziert werden konnte, veranlaßten Prusiner ein Modell zu entwickeln, welches die Replikation eines Proteins ohne Beteiligung einer Nukleinsäure beschreibt [Prusiner, Science 216 (1982), 136-44]. Nach dieser Protein-only- oder auch Heterodimer-Hypothese kann die zelluläre Isoform des PrP nur sehr langsam und unter Überwindung einer hohen Aktivierungsenergie in die pathogene Isoform konvertiert werden. Dieser Prozeß der Konformationsumwandlung wird vermutlich durch die Beteiligung von zusätzlichen, bisher noch nicht identifizierten Faktoren, durch bestimmte TSE-Mutationen von PrP oder durch exogene Applikation von PrP^{Sc}-Molekülen begünstigt [Prusiner, Brain Pathol 8 (1998), 499-513]. Entsprechend diesem Modell wird nach Ausbildung eines Heterodimers, bestehend aus PrP^{C} und PrP^{Sc}, das zelluläre PrP unter Einwirkung von PrP^{Sc} in ein PrP^{Sc}-Molekül konvertiert, was eine schnelle Ausbildung eines PrP^{Sc}-Homodimers zur Folge hat [Prusiner, Harvey Lect 87 (1991), 85-114] (vgl. Fig. 4). Dieses kann wiederum in einem autokatalytischen Prozeß die Entstehung weiterer Heterodimere exponentiell beschleunigen. Kinetischen Berechnungen zufolge ist dabei die Dissoziation des PrP^{Sc}-Homodimers nicht der geschwindigkeitsbestimmende Schritt [Eigen, Biophys Chem 63 (1996), A1-18]. Trotz intensiver Untersuchungen verschiedener Laboratorien ist es bisher noch nicht zweifelsfrei gelungen PrP-Heterodimere nachzuweisen [Priola et al., J Biol Chem 270 (1995) 3299-305; Meyer et al., J Biol Chem 275 (2000), 38081-38087]. Entsprechend diesem Modell ist eine schnelle Umwandlung der PrP-Dimere bzw. deren Instabilität zwingend notwendig.

Die Resistenz der PrP^{0/0}-Mäuse gegenüber einer Infektion mit Scrapie-Material läßt zwei mögliche Interpretationen zu [Bueler et al., Cell 73 (1993), 1339-47]: Zum einen sehen sich die Befürworter der Protein-only Hypothese in der exklusiven Bedeutung des Proteins als infektiöses Agens bestätigt. Die Anhänger der Virinohypothese dagegen argumentieren, daß das Prionprotein des Wirtes als viraler Rezeptor oder für die Verpackung der TSE-spezifischen Nukleinsäure benötigt wird [Akowitz et al., Nucleic Acids Res 22 (1994), 1101-7].
Eine schlüssige Beweisführung für die Protein-only Hypothese bei Säugern z.B. durch Generierung von rekombinanten infektiösen Prionproteinmaterial konnte bisher nicht erbracht werden [Mehlhorn et al., Biochemistry 35 (1996), 5528-37].

### Das kernabhängige Polymerisationsmodell

Das Modell der kernabhängigen Polymerisation basiert auf der Annahme eines kristallartigen Wachstums eines PrP^{Sc}-Oligomers ausgehend von einem Kristallisationskeim (vgl. Fig. 4). Diese Hypothese wurde bereits 1990 von Gajdusek beschrieben und von Caughey und Lansbury weiterentwickelt [Caughey et al., J Virol 71 (1997), 4107-10]. Dabei unterscheidet sich das Modell von der Heterodimerhypothese dadurch, daß die infektiöse Einheit von einem PrP^{Sc}-Oligomer gebildet wird, welches den Keim für die anschließende Polymerisation darstellt. Wirtseigenes PrP^{C} wird spontan und reversibel nach Einstellung von einem schnellen thermodynamischen Gleichgewicht in einen PrP^{Sc}-ähnlichen Übergangszustand umgewandelt, welcher in dem geschwindigkeitsbestimmenden Schritt der Reaktion den Kristallisationskeim angelagert wird und dessen Konformation annimmt. Die pK-Resistenz ist eine Konsequenz eines durch Polymerisation von PrP^{Sc}-Molekülen entstehenden hochmolekularen Aggregats, wobei es gelungen ist, diese Aggregate experimentell nachzuweisen und zu charakterisieren.

### Die Aufgabe der Erfindung

Der vorliegenden Erfindung liegt die Aufgabe zugrunde, Mittel bereitzustellen, die in den Prionenerkrankungen allgemein zugrundeliegenden Pathogenitätsmechanismen eingreifen. Eine weitere der Erfindung zugrundeliegende Aufgabe ist die Bereitstellung von Mitteln, die den Ablauf der Pathogenitätsmechanismen inhibieren und somit mögliche Medikamente für die Behandlung und/oder Prävention von Prionenerkrankungen darstellen.

### Lösung der Aufgabe

Erfindungsgemäß wird die Aufgabe in einem ersten Aspekt gelöst durch eine Nukleinsäure, die an ein Protein umfassend die Aminosäuren 90 bis 129 des Prionproteins bindet.

Erfindungsgemäß wird die Aufgabe in einem zweiten Aspekt gelöst durch eine Nukleinsäure, die an ein Peptid umfassend die Aminosäuren 90 bis 129 des Prionproteins bindet.

Erfindungsgemäß wird die Aufgabe in einem dritten Aspekt gelöst durch eine Nukleinsäure, die die Konversion von PrP^{C} zu PrP^{Sc} und/oder die Aggregation von PrP^{Sc} u beeinflusst.

In einer Ausführungsform ist dabei vorgesehen, dass die Konversion und/oder die Aggregation inhibiert wird.

In einer weiteren Ausführungsform ist vorgesehen, dass die Nukleinsäure eine solche gemäß dem ersten und/oder zweiten Aspekt der Erfindung ist.

Erfindungsgemäß wird die Aufgabe in einem vierten Aspekt gelöst durch eine Nukleinsäure, die die Akkumulation von PrP^{Sc}-Molekülen beeinflusst.

In einer Ausführungsform ist dabei vorgesehen, dass die Akkumulation inhibiert wird

In einer weiteren Ausführungsform ist vorgesehen, dass die Nukleinsäure eine solche gemäß dem ersten und/oder zweiten und/oder dritten Aspekt der Erfindung ist.

Erfindungsgemäß wird die Aufgabe in einem fünften Aspekt gelöst durch eine Nukleinsäure, die die Amyloidakkumulation beeinflusst.

In einer Ausführungsform ist dabei vorgesehen, dass die Amyloidakkumulation inhibiert wird

In einer weiteren Ausführungsform ist vorgesehen, dass die Nukleinsäure eine solche gemäß dem ersten und/oder zweiten und/oder dritten und/oder vierten Aspekt der Erfindung ist.

Erfindungsgemäß wird die Aufgabe in einem sechsten Aspekt gelöst durch eine Nukleinsäure, die Vermehrung und/oder die Replikation des Erregers und/oder des infektiösen Prionproteins von Erkrankungen beeinflusst, insbesondere inhibiert, wobei die Erkrankung eine neurodegenerative Erkrankung ist und insbesondere die Erkrankung ausgewählt ist aus der Gruppe, die Prionenerkrankungen, spongioforme Encephalopathien und übertragbare spongioforme Encephalopathien umfasst. Weiterhin ist in diesem Aspekt der Erfindung vorgesehen, dass die Nukleinsäure eine solche ist, die Übertragbarkeit von Prionenerkrankungen, die Prävention von Prionenerkrankungen, die Amyloidausbildung und Amyloidakkumulation beeinflusst, insbesondere inhibiert.

In einer Ausführungsform ist dabei vorgesehen, dass die Vermehrung und/oder Replikation des Erregers bzw. des infektiösen Prionproteins inhibiert wird.

In einer weiteren Ausführungsform ist vorgesehen, dass die Nukleinsäure eine solche gemäß dem ersten und/oder zweiten und/oder dritten und/oder vierten und/oder fünften Aspekt der Erfindung ist.

In einer Ausführungsform der erfindungsgemäßen Nukleinsäuren ist vorgesehen, dass die Nukleinsäure eine Sequenz umfasst, die ausgewählt ist aus der Gruppe, die SEQ ID NO:1, SEQ ID NO:2 und SEQ ID NO:3 umfasst.

In einer weiteren Ausführungsform der erfindungsgemäßen Nukleinsäuren ist vorgesehen, dass die Nukleinsäure eine Sequenz umfasst, die ausgewählt ist aus der Gruppe, die SEQ ID NO:4 bis SEQ ID NO:18 umfasst.

In einer noch weiteren Ausführungsform der erfindungsgemäßen Nukleinsäuren ist vorgesehen, dass die Nukleinsäure eine solche ist, die ausgewählt ist aus der Gruppe, die DNA, RNA, Polynukleotide, Oligonukleotide, Aptamere, Aptazyme, Spiegelmere und Intramere umfasst.

In einer Ausführungsform der erfindungsgemäßen Nukleinsäuren ist vorgesehen, dass die Derivate der Nukleinsäure wenigstens eine modifizierte Verbindungsgruppe, bevorzugterweise wenigstens eine modifizierte Verbindungsgruppe im Zuckerphosphatrückgrat und/oder wenigstens einen modifizierten Zuckerrest und/oder wenigstens eine modifizierte Base oder eine oder mehrere Kombinationen davon umfaßt.

In einer bevorzugten Ausführungsform der erfindungsgemäßen Nukleinsäuren ist vorgesehen, dass die modifizierte Base ausgewählt ist aus der Gruppe , die 5- Fluoruracil, 5-Bromuracil, 5-Chloruracil, 5-loduracil, Hypoxanthin, Xanthin, Ethenoadenosin, 4-Acetylcytosin, 5-(Carboxyhydroxylmethyl)uracil, 5-Carboxymethylaminomehtyl-2-thio-uridin, 5- Carboxymethylaminomethyluracil, Dihydrouracil, β-D-Galactosylqueosin, Inosin, N6-lsopentenyladenin, 1-Methyladenin, 1- Methylpseudouracil, 1-Methylguanin, 1-Methylinosin, 2,2-Dimethylguanin, 2-Methyladenin, 2-Ethylguanin, 3-Methylcytosin, 5-Methylcytosin, N6-Methyladenin, 7-Methylguanin, 5-Methylaminomethyluracil, 5-Methoxyaminomethyl-2-thiouracil, β-D-Mannosylqueosin, 5'-Methoxycarbonylmethyluracil, 5-Methoxyuracil, 2-Methylthio-N6-Isopentenyladenin, Uracil-5-oxyesseigsäuremethylester, Uracil-5-oxyessigsäure, Pseudouracil, Queosin, 3-Thiocytosin, 5-Methyl-2-thiouracil, 2-Thiouracil, 4-Thiouracil, 5-Methyluracil, Uracil-5-oxyessigsäuremethylester, Uracil-5-oxyessigsäure, 3(3-Amino-3-N-2-carboxypropyl)Uracil und 2,6-Diaminopurin.

In einer weiteren Ausführungsform der erfindungsgemäßen Nukleinsäuren ist vorgesehen , dass diese mindestens eine Modifikation oder Markierung am 5'- und/oder 3'-Ende, wobei bevorzugterweise die Modifikation und/oder die Markierung ausgewählt ist aus der Gruppe, die die Biotingruppe; die Digoxygeningruppe; Fluoreszenzfarbstoffe, insbesondere Fluorescein und Rhodamin; Psoralen; Thiolgruppe(n), Aminogruppe(n), Ethylenglykolgruppe(n)-, Cholesterylgruppe(n); Isotopenmarkierung; Gadolinium; Gadoliniumderivate und Technetium enthält.

In einer noch weiteren Ausführungsform der erfindungsgemäßen Nukleinsäuren ist vorgesehen, dass die modifizierte Verbindungsgruppe ausgewählt ist aus der Gruppe, die Phosphomono- oder Phosphodithioate, Alkylphosphonate, Arylphosphonate, Phosphoroamidate, Phosphattriester, bevorzugterweise P(O)-Alkylderivate; Verbindungsgruppen, in denen Sauerstoffatome in der durch die Phosphatgruppe gebildetet Brücke zwischen den Zuckern durch andere Bindungen ersetzt werden, bevorzugterweise NH-, CH₂- oder S-P-Verbindungen, bevorzugtererweise 3'-NHP(O)-(O⁻)O-5'phosphoramidate; Dephosphointernukleotidverbindungen, bevorzugterweise Acetamidat-, Carbamatverbindungen oder Peptidnukleinsäuren (PNA) umfaßt.

In einer Ausführungsform der erfindungsgemäßen Nukleinsäuren ist vorgesehen, dass der modifizierte Zuckerrest ausgewählt ist aus der Gruppe, die 2'-Azido-2'-Deoxy-, 2'-Amino-2'-Deoxy-, 2'-Fluoro-2'-Deoxy-, 2'-Chloro-2'-Deoxy, 2'-O-Methyl, 2'-O-Allyl-, 2'-C-Fluoromethyl-Gruppen und/oder Modifikationen umfaßt.

In einer bevorzugten Ausführungsform der erfindungsgemäßen Nukleinsäuren ist vorgesehen, dass die Nukleinsäure stabilisiert ist. In einer besonders bevorzugten Ausführungsform ist vorgesehen, dass mindestens ein Cytidinrest durch ein 2'-Amino-2'-deoxycytidin und/oder mindestens ein Urindinrest durch ein 2'-Amino-2'-deoxyuridin ersetzt ist.

Erfindungsgemäß wird die Aufgabe in einem siebten Aspekt gelöst durch Vektor umfassend mindestens eine der erfindungsgemäßen Nukleinsäuren.

In einer bevorzugten Ausführungsform ist der Vektor ein Expressionsvektor.

Erfindungsgemäß wird die Aufgabe in einem achten Aspekt gelöst durch eine Zelle umfassend mindestens eine der erfindungsgemäßen Nukleinsäuren und/oder mindestens einen erfindungsgemäßen Vektor.

In einer Ausführungsform ist vorgesehen, dass die Zelle eine eukaryontische Zelle ist.

In einer bevorzugten Ausführungsform ist vorgesehen, dass die Zelle eine tierische Zelle ist.

In einer ganz besonders bevorzugten Ausführungsform ist vorgesehen, dass die Zelle eine Zelle eines Säugetiers ist.

In einer noch bevorzugteren Ausführungsform ist vorgesehen, dass die erfindungsgemäße Zelle ausgewählt ist aus der Gruppe, die Saccharomyces cerevisiae, S.pombe, P. pastoris und C. elegans umfasst.

In einer noch weiteren Ausführungsform der erfindungsgemäßen Zelle ist vorgesehen, dass das Säugetier ausgewählt ist aus der Gruppe, die Mäuse, Ratten, Kaninchen, Hunde, Schweine, Affen, Hamster, Rind, Nerz, Schaf, Ziege und Menschen umfasst

Erfindungsgemäß wird die Aufgabe in einem neunten Aspekt gelöst durch Tier umfassend mindestens eine erfindungsgemäße Zelle. In einer Ausführungsform ist das Tier ein transgenes Tier.

In einer Ausführungsform ist vorgesehen, dass das Tier ausgewählt ist aus der Gruppe, die Nematoden, Fische, Mäuse, Ratten, Kaninchen, Hunde, Schweine, Hamster, Rind, Nerz, Schaf, Ziege und Affen umfasst.

Erfindungsgemäß wird die Aufgabe in einem zehnten Aspekt gelöst durch die Verwendung mindestens einer der erfindugsgemäßen Nukleinsäuren zur Herstellung eines Medikaments.

In einer Ausführungsform ist vorgesehen, dass das Medikament ein solches zur Behandlung von neurodegenerativen Erkrankungen ist.

In einer Ausführungsform ist vorgesehen, dass das Medikament ausgewählt ist aus der Gruppe, die Prionenerkrankungen, spongioforme Encephalopathien und übertragbare spongioforme Encephalopathien umfasst.

In einer Ausführungsform ist vorgesehen, dass das Medikament die Konversion von PrP^{C} zu PrP^{Sc}, die Aggregation von PrP^{Sc}, die Akkumulation von PrP^{Sc}-Molekülen, die Amyloidausbildung- und akkumulation, die Übertragbarkeit von Prionenerkrankungen und/oder die Vermehrung, insbesondere die Replikation des Erregers bzw. des infektiösen Prionproteins für eine Erkrankung beeinflusst, wobei die Erkrankung eine neurodegenerative Erkrankung ist und insbesondere die Erkrankung ausgewählt ist aus der Gruppe, die Prionenerkrankungen, spongioforme Encephalopathien und übertragbare spongioforme Encephalopathien umfasst.

In einer noch weiteren Ausführungsform ist vorgesehen, dass die Konversion von PrP^{C} zu PrP^{Sc}, die Aggregation von PrP^{Sc} , die Akkumulation von PrP^{Sc}-Molekülen, die Amyloidausbildung- und akkumulation und/oder die Vermehrung, insbesondere die Replikation des Erregers für eine Erkrankung inhibiert wird.

Erfindungsgemäß wird die Aufgabe in einem elften Aspekt gelöst durch die Verwendung eines erfindungsgemäßen Vektors in der Gentherapie.

Erfindungsgemäß wird die Aufgabe in einem zwölften Aspekt gelöst durch Verwendung mindestens einer der erfindungsgemäßen Nukleinsäuren zum Nachweis eines Prionproteins.

Erfindungsgemäß wird die Aufgabe in einem dreizehnten Aspekt gelöst durch die Verwendung mindestens einer der erfindungsgemäßen Nukleinsäuren zur Komplexbildung mit einem Prionprotein.

In bevorzugten Ausführungsformen der Verwendungen gemäß dem zwölften und dreizehnten Aspekt der Erfindung ist vorgesehen, dass das Prionprotein ausgewählt ist aus der Gruppe, die PrP^{C}, PrP^{Sc}, PrP^{res}, Prionprotein mit mehreren Oktapeptidsequenzen, Prionprotein mit unterschiedlichen Punktmutationen. In einer bevorzugten Ausührungsform ist vorgesehen, dass die Punktmutationen krankheitsrelevante Punktmutationen sind.

In einer bevorzugten Ausführungsform ist vorgesehen, dass das Prionprotein 4, 5, 6, 7, 8 oder 9 Oktapeptidsequenzen umfasst.

Erfindungsgemäß wird die Aufgabe in einem vierzehnten Aspekt gelöst durch eine Zusammensetzung, insbesondere eine pharmazeutische Zusammensetzung, die mindestens eine der erfindungsgemäßen Nukleinsäuren, einen erfindungsgemäßen Vektor und/oder eine erfindungsgemäße Zelle zusammen mit einem geeigneten Trägermaterial umfasst.

Erfindungsgemäß wird die Aufgabe in einem fünfzehnten Aspekt gelöst durch ein Verfahren zum Screenen von Kandidaten-Verbindungen, die die Wechselwirkung zwischen einem Prionprotein und einer der erfindungsgemäßen Nukleinsäuren beeinflusst, das die folgenden Schritte umfasst:
a) Bereitstellen des Prionproteins und der Nukleinsäure;
b) optional Bestimmen, ob ein Ereignis eintritt, das ausgewählt ist aus der Gruppe, die die Konversion von PrP^{C} zu PrP^{Sc}, die Aggregation von PrP^{Sc} , die Aggregation von PrP^{Sc}, die Akkumulation von PrP^{Sc}-Molekülen, die Amyloidakkumulation und/oder die Vermehrung, insbesondere die Replikation, des Erregers und/oder des infektiösen Prionproteins für eine Erkrankung beeinflusst, insbesondere inhibiert, umfasst, wobei die Erkrankung ausgewählt ist aus der Gruppe, die Prionenerkrankungen, spongioforme Encephalopathien und übertragbare spongioforme Encephalopathien umfasst;
c) Hinzufügen der Kandidatenverbindung; und
d) Bestimmen, ob eine Wechselwirkung zwischen der Nukleinsäure und dem Prionprotein beeinflusst wird und/oder es zu einer Konversion von PrP^{C} zu PrP^{Sc}, die Aggregation von PrP^{Sc}, zu einer Akkumulation von PrP^{Sc}-Molekülen, zu einer Akkumulation von PrP^{Sc}-Molekülen, zu einer Amyloidakkumulation und/oder einer Vermehrung, insbesondere einer Replikation des Erregers und/oder des infektiösen Prionproteins für eine Erkrankung kommt und/oder dies inhibiert wird, wobei die Erkrankung eine neurodegenerative Erkrankung ist, die bevorzugterweise ausgewählt ist aus der Gruppe, die Prionenerkrankungen, spongioforme Encephalopathien und übertragbare spongioforme Encephalopathien umfasst.

In einer Ausführungsform ist vorgesehen, dass das Prionprotein ausgewählt ist aus der Gruppe, die PrP^{C}, PrP^{Sc}, PrP^{res}, Prionproteine mit mehreren Oktapeptidsequenzen, bevorzugterweise mit 4 bis 9 Oktapeptidsequenzen, Prionproteine mit unterschiedlichen Mutationen, umfasst.

In einer weiteren Ausführungsform ist vorgesehen, dass die Kandidaten-Verbindung eine beliebige Verbindung ist, die beispielsweise aus einer Verbindungsbibliothek stammen kann, deren Fähigkeit getestet werden soll, ob sie die besagte Wechselwirkung zu beeinflussen, insbesondere zu inhibieren in der Lage ist.

In einer weiteren Ausführungsform ist vorgesehen, dass das Detektionsverfahren auf enzymbasierten Assays analog zu auf Antikörper basierten ELISAs beruhen kann.

Erfindungsgemäß wird die Aufgabe in einem sechzehnten Aspekt gelöst durch die Verwendung des erfindungsgemäßen Verfahrens gemäß dem fünfzehnten Aspekt der Erfindung zur Herstellung eines Medikamentes und/oder zur Ermittlung einer pharmazeutisch wirksamen Verbindung.

Erfindungsgemäß wird die Aufgabe in einem siebzehnten Aspekt gelöst durch die Verwendung der in dem erfindungsgemäßen Verfahren gemäß dem fünfzehnten Aspekt der Erfindung ermittelten Verbindung zur Herstellung eines Medikaments, wobei die Verbindung die Wechselwirkung zwischen Prionprotein und Nukleinsäure und/oder die Konversion von PrP^{C} zu PrP^{Sc}, die Aggregation von PrP^{Sc} die Akkumulation von PrP^{Sc}-Molekülen, die Amyloidakkumulation und/oder die Vermehrung, insbesondere die Replikation, des Erregers und/oder des infektiösen Prionproteins für eine Erkrankung, die Transmission des Erregers bzw. des infektiösen Prionproteins beeinflusst, wobei die Erkrankung ausgewählt ist aus der Gruppe, die Prionenerkrankungen, spongioforme Encephalopathien und übertragbare spongioforme Encephalopathien, neurodegenerative Erkrankungen umfasst.

Erfindungsgemäß wird die Aufgabe in einem achtzehnten Aspekt gelöst durch die Verwendung mindestens einer der erfindungsgemäßen Nukleinsäuren in einem in vitro-Selektionsverfahren.

Erfindungsgemäß wird die Aufgabe in einem neunzehnten Aspekt gelöst durch ein Verfahren zur Identifizierung und Isolierung von Nukleinsäuren, die in der Lage sind, an ein Zielmolekül zu binden, das die folgenden Schritte umfasst:
- Inkubieren des Zielmoleküls oder eines Teils davon mit einer Vielzahl von Nukleinsäuren, bevorzugterweise einer Nukleinsäurebibliothek, wobei die Nukleinsäuren verschiedene Sequenzen aufweisen;
- Selektieren und Isolieren jener Nukleinsäuren, die in der Lage sind, an das Zielmolekül oder einen Teil davon zu binden;
- optional Amplifizieren der isolierten Nukleinsäure und Wiederholen der ersten beiden Schritte;
- optional Bestimmen der Sequenz und/oder der Bindungsspezifität der isolierten Nukleinsäure,
wobei das Zielmolekül ein Protein oder ein Peptid umfassend eine Aminosäuresequenz entsprechend den Aminosäuren 90 bis 129 eines Prionproteins ist.

Erfindungsgemäß wird die Aufgabe in einem zwanzigsten Aspekt gelöst durch die Verwendung mindestens einer der erfindungsegmäßen zum rationalen Design von Verbindungen. In einer bevorzugten Ausführungsform ist dabei vorgesehen, die Verbindungen niedermolekulare Verbindungen sind, die aus einer Verbindungsbibliothek stammen können.

Erfindungsgemäß wird die Aufgabe in einem einundzwanzigsten Aspekt gelöst durch die Verwendung mindestens einer der erfindungsgemäßen Nukleinsäuren als diagnostisches Mittel. Hinsichtlich der Ausgestaltung von bevorzugten Ausführungsformen gilt das zur Verwendung der erfindungsgemäßen Nukleinsäuren zum Nachweis eines Prionproteins Gesagte.

In einer bevorzugten Ausführungsform ist vorgesehen, dass die Nukleinsäure in einem Komplex mit einem Prionprotein vorliegt.

Im Zusammenhang mit den verschiedenen erfindungsgemäßen Nukleinsäuren handelt es sich in bevorzugten Ausführungsformen um isolierte Nukleinsäuren.

Im Zusammenhang mit den verschiedenen erfindungsgemäßen Nukleinsäuren handelt es sich in bevorzugten Ausführungsformen um stabilisierte Nukleinsäuren.

Sofern hierin offenbart wird, dass die erfindungsgemäßen Nukleinsäuren an ein Zielmolekül binden, bedeutet dies, dass sie grundsätzlich in der Lage sind, an dieses zu binden.

Der Begriff des Prionproteins bezeichnet hierin, sofern nicht anders angegeben, das humane Prionprotein, dessen Sequenz beschrieben ist in Schatz et al. (Schatzl 1995, JMB, 245, 362-374) Weiterhin umfasst der Begriff des Prionproteins auch ein solches Prionprotein, welches eine oder mehrere, bevorzugterweise dann unterschiedliche Punktmutationen umfasst. Der Begriff Prionprotein mit einer oder mehreren unterschiedlichen Punktmutationen bezeichnet somit ein Prionprotein, das eine Mutation, d.h. ein Nukleotid oder eine Aminsoäure trägt, das bzw. die in einer Population nicht bei der Mehrheit der Individuen der Population vorhanden ist. Das Auftreten derartiger Punktmutationen kann bei einer Reihe von Erkrankungen, insbesondere neurogenerativen Erkrankungen und speziell der Jakob-Creutzfeld-Erkrankung, der fatalen familiären Insomie, Gerstmann Sträussler Scheinker Syndrom (GSS), der eine Voraussetzung für den Ausbruch der Krankheit darstellen.

Das Prionprotein kann, soweit hierin darauf Bezug genommen wird und sofern nichts anderes angeben ist, jeweils in jeglicher Glykosylierungsform vorliegen, insbesondere in der di-, mono- und nicht-glykosylierten Form. Insoweit wird auf den entsprechenden Teil der Beschreibung Bezug genommen.

Die verschiedenen Aspekte des Prionproteins und damit im Zusammenhang stehender Erkrankungen, wie sie in der Einleitung zur Veranschaulichung der zugrundeliegenden Erfindung dargestellt wurden, stellen einen Teil der Offenbarung dar. Eine Wiederholung dieser Aspekte wurde lediglich aus Gründen der Vermeidung unnötiger Wiederholungen unterlassen. Insoweit wird die Einleitung durch Bezugnahme Teil der Beschreibung der Erfindung.

Prionenerkrankungen des Menschen im Sinne der vorliegenden Erfindung sind unter anderem jene, die in Tabelle 1 dargestellt sind.

Die Beschreibung, dass die Konversion von PrP^{C} zu PrP^{Sc}, die Aggregation von PrP^{Sc}, die Akkumulation von PrP^{Sc}-Molekülen, die Amyloidausbildung-und akkumulation, die Vermehrung und/oder die Transmission, insbesondere die Replikation, des Erregers und/oder des infektiösen Prionproteins für eine Erkrankung beeinflusst wird, wobei die Erkrankung bevorzugterweise eine neurodegenrative Erkrankung ist und bevorzugtererweise ausgewählt ist aus der Gruppe, die Prionenerkrankungen, spongioforme Encephalopathien und übertragbare spongioforme Encephalopathien umfasst" ist so zu verstehen, das ein jegliches der genannten Ereignisse alleine oder in beliebiger Kombination mit einem oder mehreren der Ereignisse auftreten, oder dadurch beeinflußt oder inhibiert werden kann. Ein jeweils einzelnes Ereignis stell dar:
- die Konversion von PrP^{C} zu PrP^{Sc}
- die Aggregation von PrP^{Sc}
- die Akkumulation von PrP^{Sc}-Molekülen
- Amyloidbildung
- Transmission (Übertragbarkeit oder Übertragung)
- Prävention
- die Amyloidakkumulation und
- die Vermehrung eines Erregers oder eines infektiösen Prionproteins, die direkt oder indirekt mit einer Erkrankung verbunden ist oder zu dieser führt.

Bei der Vermehrung eines Erregers bzw. eines infektiösen Prionproteins, wobei hierin beide Begriffe synonym verwendet werden trotz ihrer wissenschaftlich geringfügig unterschiedlichen Bedeutung, soll in einer besonderen Ausführungsform die Replikation eines Erregers betroffen bzw. bezeichnet sein. Bei dem Erreger handelt es sich um den Erreger für eine bestimmte Erkrankung. Die Erkrankung kann und ist in bevorzugten Ausführungsformen eine solche, die aus der Gruppe ausgewählt ist, die Prionenerkrankungen, neurodegenerative Erkrankungen, spongioforme Encephalopathien und übertragbare spongioforme Encephalopathien umfasst. Bei den Erkrankungen kann es sich hierin prinzipiell um menschliche wie auch um tierische Erkrankungen handeln.

Für den Fall, dass die Wechselwirkung zwischen einer der erfindungsgemäßen Nukleinsäuren und einem Prionprotein, beispielsweise in einem Komplex, der entweder die Nukleinsäure oder das Prionprotein oder beide umfasst, oder deren Beeinflussung, sei es eine Förderung oder eine Hemmung derselben, bestimmt wird, kann in den verschiedenen Ausführungsformen der verschiedenen Aspekte der vorliegenden Erfindung vorgesehen sein, dass die erfindungsgemäße Nukleinsäure, das Prionprotein oder beide mit Fluoreszenzfarbstoffen markiert sind und die Beeinflussung der Wechselwirkung zwischen der Nukleinsäure und dem Prionprotein zu einer meßbaren Veränderung des Fluoreszenzsignals führt.

In einer noch weiteren Ausführungsform ist vorgesehen, dass die Nukleinsäure oder das Prionprotein als Wechselwirkungspartner mit einer fluoreszenzlöschenden Gruppe (Quencher), der jeweils andere Wechselwirkungspartner mit einer fluorophoren Gruppe (Donor) markiert ist, wobei die Wechselwirkung zwischen beiden Wechselwirkungspartnern zur Löschung der Fluoreszenzemission des Donors führt, und die Inhibition der Wechselwirkung zur Freisetzung der Fluoreszenz des Donors führt.

In einer Ausführungsform ist vorgesehen, dass die Nukleinsäure ein Aptazym ist und die Inhibition der Bindung des Prionproteins an das Aptazym durch eine Veränderung der katalytischen Aktivität des Aptazyms gemessen werden kann.

In einer noch weiteren Ausführungsform ist, dass die Wechselwirkung zwischen den Wechselwirkungspartnern, d.h. der erfindungsgemäßen Nukleinsäure und dem Prionprotein mittels Detektionsverfahren wie enzymbasierenden Assays surface plasmon resonance (SRP), fluorescence activated cell sortting FACS, fiberoptische Microarray Sensoren, Kapillarelektrophorese, quartz crystal microbalance (QCM), Kraftmessungen oder radioaktiven Messverfahren erfolgt.

Der vorliegenden Erfindung liegt die überraschende Erkenntnis zugrunde, dass es möglich ist, mit den erfindungsgemäßen Nukleinsäuren aktive Nukleinsäuren gegen einen Teil des Prionproteins zu erzeugen, der eine hohe konformationelle Plastizität [Peretz et al., J Mol Biol 274 (1997), 614-22] aufweist, was im übrigen auch dadurch bestätigt wird, dass in NMR-Studien für diesen Bereich keine definierte Sekundärstruktur identifiziert werden könnte [Zahn et al., Proc Natl Acad Sci U S A 97 (2000), 145-50]. Dieser Bereich umfaßt die Aminosäurepositionen 90 bis 129 des humanen Prionproteins. Generell scheint es eines glücklichen Griffes und der Einhaltung von spezifischen Versuchsbedingungen zu bedürfen, um aktive Nukleinsäuren wie die erfindungsgemäßen Nukleinsäuren gegen Makromoleküle, deren Struktur oder ein Teil deren Struktur sehr flexibel ist, zu erhalten. Dies ist den Erfindern der vorliegenden Anmeldung jedoch überraschenderweise gelungen. Weiterhin wurde überraschend gefunden, dass die erhaltenen erfindungsgemäßen Nukleinsäuren dabei sowohl an ein Peptid umfassend die Aminosäurereste entsprechend den Aminosäurepositionen 90 bis 129 des human Prionproteins als auch das humane Prionprotein selbst zu binden vermögen.

Nachdem Prionproteine in ihrer Funktion wenig verstanden sind, war es desweiteren überraschend festzustellen, dass unter dem Einfluss der erfindungsgemäßen Nukleinsäuren auch eine Reihe von Effekten bzw. Ereignissen beeinflußt und in der Regel inhibiert werden können. Zu diesen Effekten gehören die Konversion von PrP^{c} zu PrP^{Sc}, die Aggregation von PrP^{Sc} , die Akkumulation von PrP^{Sc}-Molekülen, die Amyloidausbildung, die Amyloidakkumulation und die Vermehrung und/oder die Transmission des Erregers bzw. des infektiösen Prionproteins, insbesondere dessen Replikation, von Prionenerkrankungen, spongioformen Encephalopathien und übertragbaren spongioformen Encephalopathien.

Infolge dieser Eigenschaften ergeben sich für die erfindungsgemäßen Nukleinsäuren eine Reihe von Anwendungsmöglichkeiten, insbesondere in der Therapeutik und der Diagnose, was in der Pathogenese der mit Prionen assoziierten neurodegenerativen Erkrankungen, die hierin auch als Prionenerkrankungen bezeichent werden und durch die Ablagerung von schwer löslichem PrP^{Sc} unter Ausbildung von polymeren PrP^{Sc}-Molekülen in Form von amyloiden Plaques charakterisiert sind [Jendroska et al., Neurology 41 (1991), 1482-90], begründet liegt.

Selektive Inhibitoren der damit einhergehenden Ereignisse und, wie die erfindungsgemäßen Nukleinsäuren, können zum einen als TSE-Therapeutika oder Diagnostika eingesetzt werden, zum anderen kann deren Anwendung dazu beitragen, den Mechanismus der Konversion von PrP^{C} zu PrP^{Sc}, die Amyloidakkumulation sowie die Funktion von PrP^{Sc} bei der Replikation des Erregers besser zu verstehen. Die inhibierende Wirkung der erfindungsgemäßen Nukleinsäuren kann in lebenden Zellen und auch in einem diese Zellen umfassenden Organismus nachgewiesen werden.

### Die PrionPeptid-Aptamere binden ihr Selektionstarget im Gesamtproteinkontext

Wie detaillierter in den Beispielen dargestellt, wurden 2'Amino-2'Desoxy-stabilisierte Aptamere gegen die Prionproteinregion AS 90-129 *in vitro* durch einen affinitätschromatographischen Prozess selektiert. Nach Abschluß der Peptidselektion (Zyklus 12) erlaubte die Primärstrukturanalyse der angereicherten RNA-Bibliothek die Einteilung der individuellen Aptamerklone in drei Sequenzfamilien, wobei Familie I mit 77% im Vergleich zur Familie II mit 14% und Familie III mit 7% den größten Anteil der erhaltenen individuellen Aptamerklone darstellt. Mit insgesamt 58% der analysierten Klone überwiegt die individuelle Sequenz #7 der Familie I. In einer Bindungsstudie konnte gezeigt werden, daß repräsentative Sequenzen der anti-Peptidaptamere das rekombinante Hamsterprionprotein, das humane Prionprotein, das Mausprionprotein und ein chimäres Maus-Hamster-Maus-Konstrukt der vollen Länge mit unterschiedlichen Affinitäten binden können (vgl. Tab. 3).

Auf der Grundlage der erhaltenen Nukleinsäuren wurden die drei als SEQ.ID.No. 1, SEQ.ID.No. 2 und SEQ.ID.No. 3 bezeichneten Konsensus-Sequenzen erhalten.

### Die PrionPeptid-Aptamere inhibieren die Akkumulation von PrP^{Sc} zu hochmolekularen Aggregaten

Der potentielle Einfluß des Prionprotein-spezifischen Aptamers #7 auf den Mechanismus der PrP^{Sc}-Entstehung sollte sowohl in *in vitro* Konversionsexperimenten, als auch in persistent prion-infizierten Neuroblastomzellen (ScN₂a,MHM₂) untersucht werden (siehe Beispiel 3). Diese Zellen exprimieren zusätzlich zum endogenen Maus-PrP ein chimäres Maus-Hamster-Maus-Konstrukt, wobei das Hamsterepitop (AS 94-131) in der Bindungsregion (AS 90-129) für die Aptamer RNA liegt.

Sowohl die partielle Resistenz von PrP^{Sc} gegenüber Proteinase K (pK) als auch die Unlöslichkeit in nichtionischen Detergentien sind wichtige Unterscheidungskriterien im Vergleich zu PrP^{C} (vgl. Tab. 2). Die pK-Resistenz korreliert wiederum mit der Menge an hochmolekularen und infektiösen PrP^{Sc}Aggregaten.

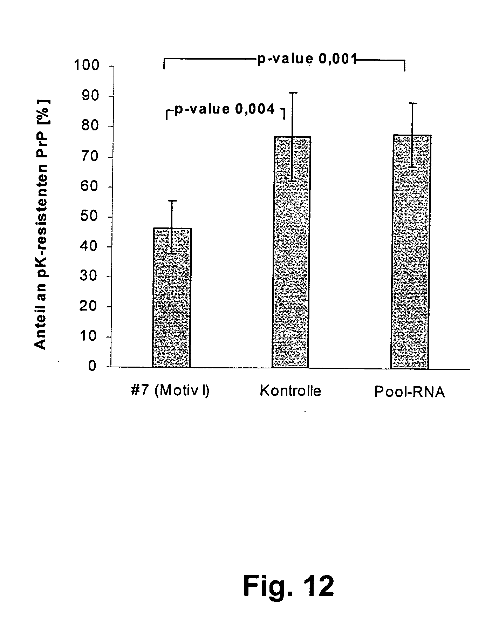

Dabei konnte im Zellkultursystem gezeigt werden, daß das Aptamer #7 den relativen Anteil an *de novo* synthetisiertem, pK-resistentem PrP^{Sc} (PrP^{res}) im Vergleich zur Pool-RNA bzw. dem PBS-Kontrollansatz statistisch signifikant reduziert (vgl. Fig. 12). Dagegen wurde kein Effekt des Aptamers auf die Biosynthese, d.h. auf die Gesamtmenge von *de novo* synthetisiertem, unlöslichen PrP^{Sc} detektiert werden. Auch in den *in vitro* Konversionsexperimenten war keine reproduzierbare Inhibition der PrP^{res}-Entstehung in Gegenwart des Aptamers nachweisbar.

Wie mit Kontrollexperimenten gezeigt werden konnte, ist der inhibitorische Effekt auf die Aggregationsbildung von PrP^{Sc} spezifisch für die erfindungsgemäßen Nukleinsäuren und insbesondere für das getestete Aptamer (#7). Für die unselektierte Pool-RNA konnte dieser Effekt nicht gezeigt werden. Die beobachtete Reduktion der Ausbildung von hochmolekularen PrP^{Sc}-Aggregaten durch die erfindungsgemäßen Nukleinsäuren spricht weder für noch gegen eine der postulierten Prionhypothesen. Während Prusiner mit seinem Modell den Schwerpunkt auf die Entstehung von PrP^{Sc} legt, beschreibt das kernabhängige Polymerisationsmodell die Entstehung von pK-resistenten Aggregaten aus einem PrP^{Sc} Kristallisationskeim. Entsprechend können die hierin offenbarten Ergebnisse sinnvoller Weise nur innerhalb des Polymerisationsmodells einordnen, die PrP^{Sc} Entstehung, wie im Prusiner-Modell betrachtet, wird durch unsere Ergebnisse nicht illustriert.

Im Verlauf der Pathogenese von spongiformen Enzephalopathien korreliert die PrP^{Sc}-Aggregatbildung (Amyloid) und die damit verbundene Akquisition der ProteinaseK-Resistenz mit Infektiösität und Neurodegeneration [Bruce et al., Neurosci Lett 102 (1989), 1-6]. Mit Blick auf diese Korrelation und den hierin offenbarten Effekten bzw. Ergebnissen ist davon auszugehen, dass die hierin offenbarten Nukleinsäuren geeignete Mittel zur Verhinderung einer Infektion bzw. einer Neurodegeneration sind, die entweder direkt therapeutisch oder im Rahmen eines geeigneten Screening-Verfahrens oder als Leitstrukturen verwendet werden können.

Wie in den Beispielen genauer erläutert werden wird, wurden die erfindungsgemäßen Nukleinsäuren erhalten vermittels der sogenannten in vitro Selektion ausgehend von einer kombinatorischen Bibliothek unterschiedlicher Nukleinsäuresequenzen. Die bei der in vitro Selektion erhaltenen, an ein Zielmolekül bindenden Nukleinsäuren werden generell und auch hierin als Nukleinsäureliganden bezeichnet. Die Techniken zur Isolierung von Nukleinsäuren durch in vitro Selektion sind dem Fachmann bekannt und in der Literatur beschrieben (Methods Enzymol. 267 (1996); Klug und Famulok, Mol. Biol. Rep. 20 (1994), 97-107; Conrad et al., Mol Div. 1 (1995), 69-78; Williams und Bartel, Nucleic Acid Mol. Biol. 10 (1996), Soukup und Breaker, Curr. Opin. Struct. Biol. 10 (2000), 318-325). Bei dem Verfahren zur in vitro Selektion wird dabei grundsätzlich so vorgegangen, dass eine Vielzahl verschiedener Nukleinsäuresequenzen (DNA, RNA und andere) mit einem Zielmolekül (engl. target) in Verbindung gebracht wird und die an das target bindende(n) Nukleinsäure(n) isoliert, wird/werden, wobei bevorzugterweise die nicht-bindenden Nukleinsäuren entfernt werden, und sodann diese Nukleinsäure(n), die gegebenenfalls in ihrer Sequenz geändert wird/werden und damit wieder eine Vielzahl von Nukleinsäuren zur Verfügung gestellt wird, erneut mit dem target in Kontakt gebracht wird. Diese Schritte können mehrfach wiederholt werden.

Nukleinsäuren oder Nukleinsäureliganden im Sinne dieser Erfindung umfassen bevorzugt DNA, RNA oder chemisch modifizierte Formen von DNA oder RNA. Noch bevorzugter gehören die Nukleinsäureliganden zu einer der Klassen der Aptamere, Aptazyme, Ribozyme, Intramere, natürliche Nukleinsäureliganden oder sind natürlich vorkommende Protein-bindende Nukleinsäuren. Besonders bevorzugt sind dabei die Aptazyme, Intramere, Spiegelmere und die Aptamere, von den dreien wiederum insbesondere die Gruppe der Aptamere und Intramere. Unter natürlichen Nukleinsäuren sollen hierin auch solche Nukleinsäuren verstanden werden, sei es als Ausgangmaterialien (teilweise oder vollständig) für in vitro Selektionsverfahren oder sei es als erfindungsgemäße Nukleinsäuren, die eine Sequenz aufweisen, die im Transkriptom einer Zelle bzw. eines Organismus enthalten ist, wobei unter Transkriptom hierein insbesondere die Gesamtheit der exprimierten Nukleinsäuren verstanden wird. Im Falle der Verwendung von natürlichen Nukleinsäuren ist besonders bevorzugt, statt randomisierten Bibliotheken in Selektionsprozessen natürliche Bibliotheken einzusetzen, wie z.B. cDNA-Fragmente transkribierter RNAs.

Unter vitro Selektion soll hierin insbesondere auch der Prozess verstanden werden durch den die erfindungsgemäßen Nukleinsäureliganden, bevorzugt aus der Gruppe der Aptamere, der Intramere, der Aptazyme oder der Ribozyme, noch bevorzugter aus der Gruppe der Aptamere und Aptazyme, durch in vitro Selektion aus kombinatorischen Bibliotheken von Nukleinsäuren mit unterschiedlichen Sequenzen isoliert werden können.

Unter Aptameren (Ellington uns Szostak, Nature 346 (1990), 818-822) sind im Sinne dieser Erfindung einzelsträngige Nukleinsäureliganden zu verstehen die aus kombinatorischen Bibliotheken von randomisierten Nukleinsäuren.durch in vitro Selektion isoliert werden. Dieser Prozess wird manchmal auch als SELEX ("systematic evolution of ligands by exponential enrichment") bezeichnet (Tuerk und Gold, Science 249 (1990), 505-519). Diese aus ssDNA oder ssRNA bestehenden Aptamere besitzen sehr hohe Affinitäten und Spezifitäten für ihre Antigene. Bis dato wurden Nukleinsäureliganden für Metallionen, organische Verbindungen, Peptide, Proteine, oder sogar komplexe Strukturen wie Viren und Zellen isoliert (Übersichtsartikel: Gold et al., Annu. Rev. Biochem. 64 (1995), 763-797; Ellington und Conrad, Biotechnol. Annu. Rev. 1 (1995), 185-214; Famulok, Curr. Opin. Struct. Biol. 9 (1999), 324-329, Hofmann, 1997). Das hohe Potential der Technologie liegt in dem rein *in vitro* stattfindenden Prozess der Aptamerselektion. Die Nukleinsäureliganden werden aus kombinatorischen Bibliotheken von bis zu 10¹⁵ individuellen Sequenzen durch wiederholte Zyklen aus Kontakt mit dem Antigen, Abtrennung aller nicht bindenden Nukleinsäuren und enzymatischer Amplifikation der mit dem Zielmolekül interagierenden Moleküle angereichert.

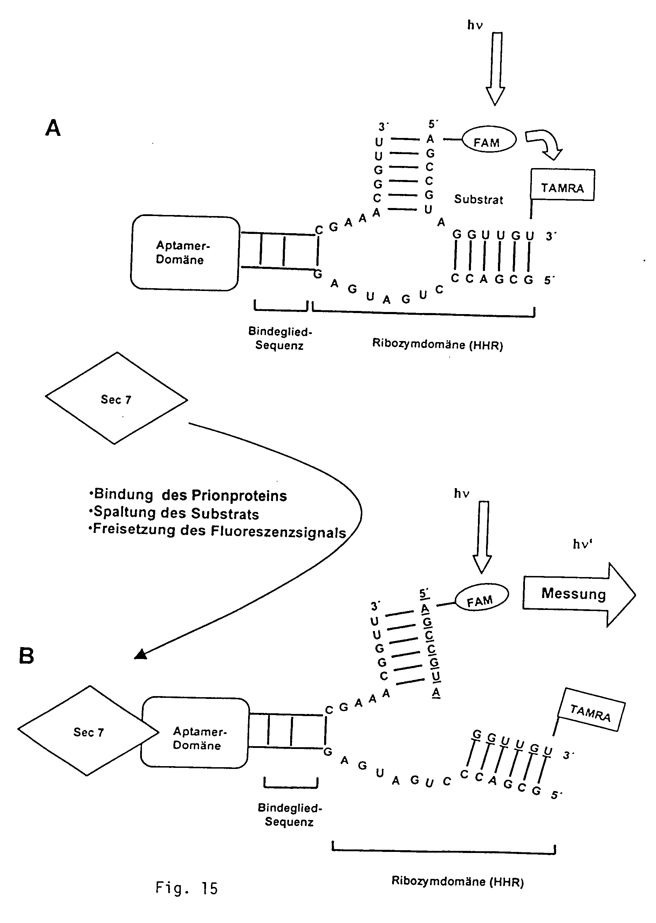

Aptazyme im Sinne der vorliegenden Erfindung, sind Hybridmoleküle aus Ribozymen und Aptameren und bestehen dementsprechend aus einer katalytischen und einer ligandenbindenden Nukleinsäuredomäne. Dabei wird die Aktivität des Ribozyms durch die Bindung des Liganden an die ligandenbindende Nukleinsäuredomäne (allosterisches Zentrum) reguliert, d.h. aktiviert oder inhibiert. Die Aptazymtechnologie ist dem Experten bekannt und beispielsweise beschrieben in Soukup und Breaker, Curr. Opin. Struct. Biol. 10 (2000), 318-325. Dabei können Aptazyme durch rationales Design aus einem Ribozymteil und dem allosterischen Zentrum modular aufgebaut werden oder de novo durch in vitro Selektion isoliert werden. Beispielsweise wurde ein Aptazym aus einem Hammerhead-Ribozym, einem kleinen katalytischen Motiv, das RNA-Sequenzen sequenzspezifisch spalten kann (Forster und Symons, Cell 49 (1987), 211-220; Haseloff und Gerlach, Nature 334 (1988), 585-591), und einer Aptamerdomäne, die spezifisch an ATP bindet, so konstruiert, dass die Bindung von ATP zu einer allosterischen Inhibition der katalytischen Aktivität führte (Tang und Breaker, Chem. Biol. 4 (1997), 453-459, Tang und Breaker, Nucleic Acid Res. 26 (1999), 4222-4229). Andererseits konnten auch Aptazyme durch in vitro Selektion aus kombinatorischen Bibliotheken von unterschiedlichen Nukleinsäuresequenzen isoliert werden, die eine neuartige Bindungsdomäne für einen Liganden enthielten (Robertson und Ellington, Nat. Biotechnol. 17 (1999), 62-66,Koizumi et al., Nat. Struct. Biol. 6 (1999), 1062-1071). Aptazyme im Sinne dieser Erfindung können beispielsweise solche sein, die eine Liganden-Bindungsstelle für Prionproteine enthalten. Derartige Aptazyme können auf der Grundlage der erfindungsgemäßen Nukleinsäuren dadurch hergestellt werden, dass diese mit einem Ribozymteil und dem allosterischen Zentrum modular aufgebaut werden. Eine schematische Darstellung eines Aptazyms ist in Abbildung 15 dargestellt

Unter natürlichen Nukleinsäureliganden sollen hierin in Ergänzung zu dem vorstehend Gesagtem auch solche Nukleinsäuren, Nukleinsäureliganden oder Teile davon, d.h. Nukleinsäuremotive, verstanden werden, die als solche in den Genomen von Organismen kodiert werden und die Fähigkeit besitzen an ein Zielprotein zu binden. Beispiele sind unter vielen anderen die HIV-Tar-RNA, die an das HIV Tat-Protein bindet, oder das HIV-RRE-RNA, die an das HIV-REV-Protein bindet. Dem Fachmann sind eine Fülle solcher natürlicher Nukleinsäureliganden bekannt. Bislang unbekannte Nukleinsäureliganden für bestimmte Zielproteine können auch aus kombinatorischen Bibliotheken von natürlichen Nukleinsäuren unterschiedlicher Sequenz, die beispielsweise die genomische RNA eines Organismus, eines Organs oder einer Zelle repräsentieren, durch in vitro Selektion isoliert werden. Die Herstellung solcher kombinatorischer Bibliotheken ist dem Fachmann bekannt und beispielsweise beschrieben in Singer et al., Nucleic Acid Res. 25 (1997), 781-786. Auf diese Weise konnten beispielsweise genomische DNA-Sequenzen, die den Transkriptionsfaktor TFIIIA von Xenopus laevis binden, isoliert werden (Kinzler und Vogelstein, Nucleic Acid Res. 17 (1989), 3645-3653). In einem anderen Experiment konnten aus mRNA-Fragmenten aus dem Transkriptom des Bakteriums E.coli Liganden für das Bakteriophagen MS2 Protein isoliert werden (Shtatland et al., Nucleic Acids Res. 28 (2000), E93). Dabei wurden überraschenderweise neue, bislang unbekannte Interaktionen beispielsweise mit den mRNAs des rffG-Gens oder des ebgR-Gens gefunden. Dies zeigt, dass neue Nukleinsäure-Liganden für Proteine aus Bibliotheken, die aus dem Transkriptom eines Organismus aufgebaut sind, isoliert werden können.

Wie bereits hierin ausgeführt, können die erfindungsgemäßen Nukleinsäuren als Intramere ausgebildet vorliegen. In dieser speziellen Ausgestaltungsform können sie sowohl für die therapeutische als auch diagnostische Anwendung von Vorteil sein, da PrPc internalisiert werden und die Konversion zu PrPsc im Zytoplasma stattfinden kann. Unter Intrameren sollen hierin solche Aptamere oder natürliche Nukleinsäuresequenzen oder natürliche Nukleinsäureliganden verstanden werden, die neben der reinen Bindung ihres Liganden spezifisch für den Einsatz im intrazellulären Milieu konstruiert worden sind. Ganz allgemein wird dabei so vorgegangen, dass zu den erfindungsgemäßen Nukleinsäuren weitere Sequenzen hinzugefügt werden, die dazu führen, dass die erfindungsgemäße Nukleinsäure in einem intrazellulären Milieu stabil ist und/oder exprimiert wird. Typischerweise können derartige zusätzliche Nukleinsäuren, die den vorstehend genannten Anforderungen genügen bzw. die entsprechenden erforderlichen Eigenschaften verleihen, bereits dadurch angefügt werden, dass die erfindungsgemäße Nukleinsäure in ein Expressionssystem eingefügt wird. Beispielsweise können durch Verwendung rein viraler Expressionssysteme durch Anfügen von viralen RNA-Sequenzen und der Verwendung von viralen Promotoren eines kombinierten Systems aus dem Vacciniavirus und dem T7-Bakteriophagen Aptamere stabilisiert, mit hoher Transkriptionseffizienz und lokalisiert im Zytoplasma durch virale Polymerasen transkribiert werden und dort ihre Zielmoleküle binden (Blind et al., Proc. Natl. Acad. Sci. USA 96 (1999), 3606-3610). Unter Intrameren sollen hierin insbesondere solche in vitro selektierten Aptamere verstanden werden, die durch Stabilisierung, Lokalisation, Expression, Anfügen natürlicher oder nicht-natürlicher Nukleinsäuresequenzen, chemische Modifikationen oder andere Maßnahmen zum Zwecke der funktionalen Modulation der Aktivität eines intrazellulären Zielmoleküls optimiert und eingesetzt werden. Andere Expressionssysteme, die zur Transkription von Intrameren verwendet werden können, sind beispielsweise eukaryontische RNA-Polymerase-II-abhängige Systeme. So wurden Aptamere gegen einen am Splicing in Drosophila melanogaster beteiligtes Protein (B52) über einen Hitzeschockpromotor induzierbar in transgenen Fliegen exprimiert (Shi H. et al., Proc. Natl. Acad. Sci USA 96 (1999), 10033-10038). Ebenso sind viele Expressionssysteme bekannt, die für die Transkription intrazellulärer Ribozyme benutzt wurden. Beispielsweise wurden RNA-Polymerase-III-Promotoren für die Transkription der RNA-Ribozyme eingesetzt. Zu diesem Zweck wurde die funktionalen Ribozym-Sequenzen meist im Kontext von Teilen kleiner natürlicher RNA-Moleküle in cis eingesetzt. Durch Anfügen von kompakten Sequenzen aus tRNA oder U6-RNA konnten sehr hohe Transkriptionsausbeuten von Ribozymtranskripten erzielt werden (Lee et al., Gene Ther. 2 (1995),377-384, Zouh et al., Thompson et al., Nucleic acid res. 23, 2259-2268; Bertrand et al., RNA 3 (1997), 75-88). Weitere auf Polymerase-III-Promotoren, die sich für die Expression von RNA in Zellen eignen sind dem Fachmann bekannt und in der Literatur beschrieben (Übersichtsartikel: Couture und Stinchcomb, TIG 12 (1996), 510-514; Rossi, Tibtech 13 (1995), 301-305; Bramlage et al., Tibtech 16 (1998), 434-438).. Sie sind für die Transkription vieler kleiner RNA-Moleküle in eukaryontischen Zellen verantwortlich und in allen Zelltypen aktiv. Zudem sind sie in homologen RNA-Transkriptionseinheiten ubiquitär in allen eukaryontischen Spezies zu finden. Pol-III-Promotoren steuern die Transkription kleiner RNA wie tRNA, snRNA, snoRNA, 5S rRNA, oder kleiner viraler RNA, wie der adenoviralen VA-RNA. Ein weiterer Vorteil der RNA-Pol III-Promotoren sind ihre äußerst hohen Transkriptionsaktivitäten. Natürliche Transkripte akkumulieren in Zellen auf bis zu 10⁵ bis 10⁶ Moleküle pro Zelle. Beispiele für RNA-Polymerase III-Promotoren sind die Promotoren von 5S-RNA-Genen (Typ 1) (Specht et al., Nucleic Acid Res. 19 (1991), 2189-2191), tRNA-Promotoren (Typ 2) (Thompson et al., Nucleic Acid Res. 23 (1995), 2259-2268; Sullenger et al., J. Virol. 65 (1991), 6811-6816) U6 snRNA (Typ 3) (Das et al., EMBO J. 7 (1988), 503-512; Lobo und Hernandez, Genes Dev. 58 (1989), 55-67) oder andere nicht unter die Klassifizierung 1 bis 3 fallende Pol-III-Promotoren, wie z.B. 7SL-RNA-Promotoren (Bredow et al., Gene 86 (1989), 217-225). Für die Expression von RNA-Molekülen wurden bisher einige dieser Promotoren, unter anderem tRNA, U6 snRNA- oder VA1-RNA-Promotoren verwendet (Übersichtsartikel: Rossi, Tibtech 13 (1997), 301-305; Bramlage et al., Tibtech 16 (1998), 434-438).

Ein weiteres geeignetes Expressionsystem stellt das sogenannte Pol III - Expressionssystem dar. Das Pol III-Expressionssystem umfasst dabei eine Nukleinsäuresequenz, die in 5'- 3'- Richtung die folgenden Elemente umfasst:
ein C1-Motiv,
eine A1-Box,
ein C2-Motiv,
eine A3-Box, und
einen Terminator,
wobei das C1-Motiv und das C2-Motiv zusammen eine Helix ausbilden;
die A1-Box k Basen umfasst, wobei k unabhängig von I und m eine ganze Zahl von 0 bis 100 ist;
die A2-Box I Basen umfasst, wobei I unabhängig von k und m eine ganze Zahl von 0 bis 100 ist, und
die A3-Box m Basen umfasst, wobei m unabhängig von k und l eine ganze Zahl von 0 bis 20 ist.

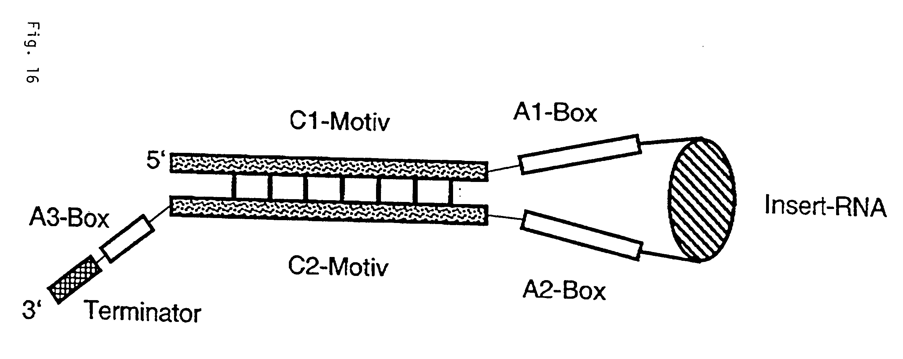

Ein derartiges Pol III-Expressionssystem ist in Fig. 16 dargestellt.

Um die Möglichkeiten der Aptamertechnologie intensiver nutzen zu können, können chemische Derivate der Desoxyribonuklein- und Ribonukleinsäuren entwickelt werden. Beispielsweise können die Nukleinsäuren gegen Degradation durch Nukleasen stabilisiert werden. Eine derartige Stabilisierung kann an verschiedenen Stellen des Nukleinsäuremoleküls erfolgen. So ist eine Modifizierung der Verbindungsgruppe zwischen den Nukleobasen, d.h. der Phosphodiesterbindung, aber auch des Zuckerrestes oder der Basen oder Kombinationen dieser Maßnahmen möglich. Die unterschiedlichen Möglichkeiten chemische veränderte Nukleinsäuren herzustellen sind dem Fachmann bekannt und ausführlich in der Literatur beschrieben (Übersichtsartikel: Uhlmann E. und Peyman A., Chem Reviews 90 (1990), 543-584; Eaton B.E. und Pieken W.A., Annu Rev Biochem 64 (1995), 837-863; Eaton B. E., Curr Opin Chem Biol 1 (1997), 10-16). Insbesondere bevorzugt sind die mit der *in vitro* Transkriptionsreaktion des Selektionsprozesses kompatiblen 2'-Amino- und 2'-Fluoro-Deoxyribonukleotide. In Anwesenheit von Nukleasen erhöhen sich die Halbwertszeiten der modifizierter Moleküle gegenüber natürlichen Nukleinsäuren von Minuten auf mehrere Stunden (Lee S.-W. und Sullenger B.A., Nat Biotechnol 15 (1997), 41-45; Kubik et al., J. Immunol. 159 (1997), 259-267; Pagratis et al., Nat. Biotechnol. 15 (1997), 68-73). Es konnte auch gezeigt werden, dass die nachträgliche Modifikation der Zuckerreste von Purinen mit 2'-O-Methylgruppen und das Anfügen von kurzen Phosphorothioatgruppen an den 5'- und 3'-Enden die Stabilität von RNA-Aptameren in biologischen Medien deutlich erhöhen können (Green et al., Chem. Biol. 2 (1995), 683-695). Die Ribonukleinsäureliganden erreichen Halbwertszeiten von bis zu 72 Stunden. Für die erfindungsgemäßen 2'-aminomodifizierten PrP-Aptamere (2'-Position der Pyrimidine des Riboseringes) konnte eine Stabilität über 48 Stunden in 10%FKS gezeigt werden.

Durch Einführung von chemischen Verbindungsgruppen können Nukleinsäureliganden strukturell stabilisiert werden. Ein Beispiel ist die strukturelle Stabilisierung von zwei kurzen Oligonukleotiden aus dem minimalen RBE (Rev binding element) durch eine verbindende Stilbendicarboxyamid-Brücke (Nelson et al, Biochemistry 35 (1996), 5339-5344). Das RBE in der genomischen RNA des HIV-Virus bindet an das virale Rev-Protein. Rev ist daraufhin verantwortlich für den Transport der viralen RNA aus dem Zellkern in das Zytoplasma. Ohne die chemische Verknüpfung waren die beiden Oligonukleotide nicht in der Lage thermisch stabile Strukturen über Basenpaarung auszubilden. Mit der Stilbendicarboxyamid-Brücke war die Struktur stabilisiert und band das Rev-Protein mit gleichen Affinitäten wie das virale RBE. Diese Methode ist insbesondere nützlich zur Herstellung von stark verkürzten funktionalen Nukleinsäureliganden.

Die erfindungsgemäßen Nukleinsäuren sind grundsätzlich und auch bei den verschiedenen möglichen und insbesondere den hierin offenbarten Verwendungen oder Anwendungen nicht auf die spezifischen offenbarten Sequenzen beschränkt. Vielmehr ist es im Rahmen der Fähigkeiten der Fachleute auf diesem Gebiet, ausgehend von den konkret offenbarten Nukleinsäuren, diese zu modifizieren. Derartige Nukleinsäuren werden hierin auch als modifizierte Nukleinsäuren bezeichnet. Wann immer hierin oder in den Ansprüchen von den erfindungsgemäßen Nukleinsäuren und deren Anwendung oder Verwendung die Rede ist, beinhaltet dies auch gleichzeitig immer die modifizierten Nukleinsäuren und deren Anwendung oder Verwendung. Im Rahmen einer derartigen Modifizierung können Nukleobasen chemisch modifiziert werden oder auch einzelne oder mehrere der Nukleobasen ausgetauscht oder deletiert werden. Die Herstellung von Nukleinsäuren, bei denen Nukleobasen ausgetauscht oder deletiert werden und trotzdem die gleichen Bindungseigenschaften wie die ursprünglichen Nukleinsäureligenden aufweisen sind dem Fachmann bekannt und in der Literatur beschrieben. Ein Beispiel ist die Erzeugung von mutierten und verkürzten Derivaten eines Aptamers gegen den Transkriptionsfaktor NFκB (Lebruska und Maher, Biochemistry 38 (1999), 3168-3174). Um Mutanten eines Aptamers (Aptamer 3) zu isolieren, die wie das ursprüngliche Aptamer 3 an den Transkriptionsfaktor NFkB binden, wurde eine Bibliothek von Mutanten hergestellt, die an jeder Position die korrekte Base mit einer Wahrscheinlichkeit von 0,7 enthielt und die übrigen drei Basen mit einer Wahrscheinlichkeit von je 0,1. Durch in vitro Selektion wurden Mutanten isoliert, die trotz Austausch von Nukleobasen immer noch an den Transkriptionsfaktor banden. Um Deletionen zu erzeugen, wurde das ursprüngliche Aptamer durch alkalische Hydrolyse partiell gespalten und in Bindungsstudien solche Fragmente isoliert, die trotz der Deletionen noch an den Transkriptionsfaktor NFκB banden. Auf diese Weise konnten verkürzte Formen des ursprünglichen Aptamers hergestellt werden. Es können aber auch zu den erfindungsgemäßen Sequenzen, wie bereits im Zusammenhang mit den Aptazymen und Intrameren ausgeführt, weitere Nukleotide hinzugefügt werden. Insoweit stellen die konkreten Sequenzen der hierin offenbarten Nukleinsäuren einen Rahmen dar, von dem ausgehend weitere Sequenzen denkbar sind und der nur dadurch begrenzt wird, dass auch die solchermaßen modifizierte Nukleinsäure nach wie vor an das Prionprotein binden und bei bevorzugten Ausführungsformen auch die PrPsc-Akkumulation zu hochmolekularen infektiösen Aggregaten, die Konversion von PrPc zu PrPSc, die Amyloidausbildung, die Amyloidakkumulation und/oder die Vermehrung, insbesondere der Replikation des Erregers bzw. des infektiösen Prionproteins für eine Krankheit moduliert, insbesondere inhibiert, wobei die Krankheit eine neurodegenerative Erkrankung ist und insbesondere die Erkrankung ausgewählt ist aus der Gruppe, die Prionenerkrankungen, spongioforme Encephalopathien und übertragbare spongioforme Encephalopathien umfasst.

Die Erfindung betrifft in einem weiteren Aspekt ein Expressionssystem, das mindestens eine oder mehrere der erfindungsgemäßen Nukleinsäuren oder Modifikationen davon umfasst. Unter Expressionssystem sollen hierin insbesondere Vektoren verstanden werden, die geeignete Expressionskassetten für die erfindungsgemäßen Nukleinsäuren enthalten und die Einschleusung und/oder die Expression der erfindungsgemäßen Nukleinsäuren in Zellen ermöglichen. Besonders bevorzugt sind hier eukaryontische Zellen. Geeignete Vektoren zur Einschleusung der Gene sind dem Fachmann bekannt. Sie können bloße DNA-Vektoren wie Plasmide, Cosmide, BACs ("bacterial artificial chromosomes"), YACs ("yeast artificial chromosomes"), MACs ("mammalian artificial chromosomes") umfassen. Desweiteren sind beispielsweise virale Vektoren, wie Sindbisviren, Vacciniaviren, Adenoviren, AAV oder Retroviren möglich. Desweiteren betrifft die Erfindung Expressionskassetten, die zumindest eine der erfindungsgemäßen Nukleinsäuren bzw. die Gene für die erfindungsgemäßen Nukleinsäureliganden, bevorzugt RNA-Liganden, umfassen, wie die in Beispiel 4 beschriebene (vgl. auch Blind et al., Proc Natl. Acad. Sci. USA 96 (2000), 3606-3610; PCT/EP/00/02727). Andere Beispiele für Expressionskassetten sind beschrieben in Good et al., Gene Ther. 4 (1997), 45-54 oder Bertrand et al., RNA 3 (1997), 75-88. Dabei umfassen derartige Expressionskassetten typischerweise geeignete Promotoren zur Expression der Nukleinsäureliganden und können zusätzlich zu den Nukleinsäureliganden noch für Sequenzen kodieren, die dem Hybridmolekül aus Nukleinsäureligand und zusätzlichen Sequenzen intrazelluläre Stabilität verleihen oder seine intrazelluläre Lokalisierung steuern (Blind und Famulok, DE 100 46 913.2).

Die Verwendung der erfindungsgemäßen Nukleinsäuren kann bei der Therapie sowohl in der direkten Verabreichung derselben erfolgen als auch im Rahmen der Gentherapie. Im Falle der Gentherapie werden dabei die die erfindungsgemäßen Nukleinsäuren enthaltenden Vektoren, Expressionssystem und Expressionskassetten von besonderer Bedeutung sein. Bei der gentherapeutischen Verwendung der erfindungsgemäßen Nukleinsäuren ist dabei der grundsätzliche Anwendungsaspekt der, dass eine die erfindungsgemäßen Nukleinsäuren enthaltende Expressionskassette durch einen geeigneten Vektor in Zellen eines eukaryontischen Organismus, insbesondere in humane Zellen, eingeschleust werden. Von der eingeschleusten Expressionskassette können die erfindungsgemäßen Nukleinsäuren dann in den Zellen exprimiert werden um dort die Akkumulation von PrPsc-Molekülen, Aggregation von PrPSc die anderen hierin beschriebenen, von den erfindungsgemäßen Nukleinsäuren ausgehenden Effekten zu inhibieren. Als Vektoren für gentherapeutische Ansätze können freie DNA, in Trägermaterialien wie kationische Liposomen verpackte DNA, die weiter unten näher beschrieben sind, oder virale Vektoren wie Adenoviren, adeno-assoziierte Viren, Retroviren, Herpesviren, Polyomaviren, Papillomaviren oder Vacciniaviren verwendet werden (Übersichtsartikel: Walther und Stein, Drugs 60 (2000), 249-271; Buchschacher und Wong-Staal, Blood 95 (2000), 2499-2504; Krauzewicz und Griffin, Adv. Exp. Med. Biol. 465 (2000), 73-82; Virtanen et al., Adv. Exp. Med. Biol. 465 (2000), 423-429). Geeignete Vektoren für die erfindungsgemäße Nukleinsäuresequenz sind den Fachleuten bekannt. Als freie DNA-Vektoren können unter anderem Plasmide und Cosmide verwendet werden, die durch Elektroporation, Lipofektion oder CaPO₄-Präzipitation in die Zellen eingeschleust werden (Übersichtsartikel: Gregoriadis, Pharm. Res. 15 1998, 661-670). Eine Erweiterung dieser Methode ist die Verwendung episomal replizierender Plasmide, die den "origin of replication" des Ebstein-Barr Virus (OriP) tragen und das EBNA-1-Antigen exprimieren. Diese Vektoren replizieren extrachromosomal in Primaten- und Hund-Zelllinien und können dort dauerhaft persistieren (Yates et al., Nature 313 (1985), 812-815; Chittenden et al., J. Virol. 63 (1989), 3016-3025. Eine andere Methode, fremdes genetisches Material in eukaryontischen Zellen dauerhaft zu replizieren, ist die Verwendung von sogenannten Minichromosomen. Diese großen DNA-Moleküle tragen wie natürliche Chromosomen Zentromer- und Telomersequenzen und werden in der Mitose dupliziert und an die Tochterzellen weitergegeben. Ihre Größe (im Megabasenbereich) erlaubt zudem die Klonierung sehr großer DNA-Fragmente von mehreren 100 000 Basen. Minichromosomen wurden für Bakterien-, Hefe- und Säugerzellen entwickelt (YACs und BACs, siehe: Grimes und Cooke, Hum. Mol. Genet. 7 (1998), 1635-1640; Amemiya et al., Methods Cell. Biol. 60 (1999), 235-258; Brown et al., Trends Biotechnol. 18 (2000), 218-223. Die hier aufgeführten Vektoren sind nicht limitierende Beispiele für Vektorsysteme, die in der Literatur beschrieben und den Fachleuten bekannt sind.

Häufig benutzte virale Vektoren sind Retroviren, deren RNA-Genom nach der reversen Transcription als DNA stabil in das Genom des Wirts integriert. Am häufigsten werden auf MoMuLV basierende Vektoren eingesetzt, die allerdings nur proliferierende Zellen infizieren können. Daher wurden auch auf Lentiviren (u.a. HIV) basierende Systeme entwickelt, mit denen auch sich nicht-teilende Zellen infiziert werden können (Übersichtsartikel: Miller, Hum Gene Ther. 1 (1990), 5-14; Gordon und Anderson, Curr. Opin. Biotechnol. 5 (1994), 611-616). Eine andere häufig verwendete Klasse sind adeno-assoziierte Viren (AAV). Diese ssDNA-Viren zeichnen sich unter anderem durch den Vorteil aus, dass sie genetisches Material an einer definierten Stelle im Chromosom 19 integrieren können (Übersichtsartikel: Grimm und Kleinschmidt, Hum. Gene. Ther. 10 (1999), 2445-2450).

Die direkte Verabreichung, gegebenenfalls aber auch die Verabreichung im Rahmen der Gentherapie, der erfindungsgemäßen Nukleinsäuren kann dabei vermittels aller galenischer Techniken und Zusammensetzungen, die eine oder mehrere der erfindungsgemäßen Nukleinsäuren in ihren verschiedenen Ausführungsformen umfassen, erfolgen. Beispielhaft sei hier auf Lösungen, Pulver, Tabletten, Gele und dergleichen verwiesen. Dabei wird typischerweise die erfindungsgemäße Nukleinsäure zusammen mit einem Stabilisator, Adjuvanz, pharmazeutisch akzeptablen Träger, Konservierungsmittel, Verdünnungsmittel, Träger oder einer Kombination davon hergestellt und/oder verabreicht. Methoden zur Einschleusung der erfindungsgemäßen Nukleinsäuren in Zellen sind dem Fachmann bekannt. Die erfindungsgemäßen Nukleinsäuren können direkt beispielsweise als liposomale Formulierungen verwendet werden. Transfectionsprotokolle mit kationischen Liposomen sind beschrieben in Ausubel et al. (Current Protocolls in Molecular Biology (1991), Wiley Interscience , New York) oder Sambrook et al. (Molecular cloning - a laboratory manual, 2^{nd} Ed. (1989), Cold Spring Harbor Laboratory Press, Cold Spring Harbor). Detaillierte Beschreibungen über die Art von kationischen Amphiphilen (Liposomen), die sich auch für die in vivo Verabreichung von therapeutischen Nukleinsäuren eignen sind beschrieben in Felgner et al. (Proc Natl Acad Sci USA (1987), 7413-7417), Behr et al., Proc. Natl. Acad Sci USA 86 (1994), Epand et al., (US 5,283,185) oder Lee et al. (US 5,925,628). Andere Transfektionsreagentien für Nukleinsäuren sind beispielsweise Dendrimere (Tang et al., Bioconjugate Chem 7 (1996), 703) oder andere polykationische Polymere (z.B. beschrieben in Illum, US 5,744,166). Eine weitere Möglichkeit zur Einführung von Nukleinsäuren in Zellen sind Hybridmoleküle aus der Nukleinsäure und kurzen Signalpeptiden, die natürlicherweise z.B. in der basischen Domäne des HIV Tat-Proteins oder der Helix 3 des Antennapediaproteins vorkommen. Diese Signalpeptide können Makromoleküle über einen bislang noch nicht im Detail verstandenen Prozeß direkt durch die Zytoplasmamembran in das intrazelluläre Kompartiment einschleusen. Die Verendung solcher Signalpeptide wurde beispielsweise beschrieben von Troy und Shelanski (US 5929042) oder von Lin. und Hawiger. (US 5,807,746). Die angeführten Reagenzien dienen der Beschreibung der vielfältigen Möglichkeiten zur Einschleusung von Nukleinsäuren in Zellen, die dem Fachmann bekannt sind, und sollen hier als nicht limitierende Beispiele verstanden werden.

Eine weitere Anwendung der erfindungsgemäßen Nukleinsäuren liegt im diagnostischen Bereich. Nukleinsäureliganden können wie Antikörper in einer Vielzahl von diagnostischen Applikationen weingesetzt werden (Übersichtsartikel: Hesselberth et al., J Biotechnol 74 (2000), 15-25; Brody und Gold, Biotechnol 74 (2000), 5-13; Jayasena Clin Chem 45 (1999), 1628-1650; Osborne et al., Curr Opin Chem Biol 1 (1997), 5-9). Durch die Bindung der erfindungsgemäßen Nukleinsäuren an die Zielstruktur, d.h. das Prionprotein, kann dieses markiert werden. Trägt die erfindungsgemäße Nukleinsäure ihrerseits ein Markierung, kann diese und damit der Komplex aus PrP und erfindungsgemäßer Nukleinsäure nachgewiesen werden. Geeignete Markierungen sind den Fachleuten auf dem Gebiet bekannt und umfassen, unter anderem, jene, die aus der Gruppe ausgewählt sind, die radioaktive Markierungen, Markierungen mit Fluoreszenzfarbstoffen, Markierung mit Gruppen wie Biotin oder Digoxygenin oder Markierungen mit Enzymen wie beta-Galaktosidase umfasst. Geeignete Fluoreszenzfarbstoffe für die Markierung von Nukleinsäuren sind den Fachleuten bekannt und umfassen u.a. AMCA-X, Fluorescein, Rhodamin, Cy3, Cy 5, Cy 5.5, HEX, D-AMCA, Tetramethylrhodamin oder Texas Red. Der Nachweis des besagten Komplexes mit andern Nukleinsäure/Proteinkomplexen kann beispielsweise durch FACS (Ringquist und Parma , Anal Chem. 70 (1998), 3419-3425), fiberoptische Microarray Sensoren (Lee und Walt, Anal Biochem 282 (2000), 142-146), Kapillarelektrophorese (German I., Anal Chem 70 (1998), 4540-4545), intermolekulare Kraftmessungen (Moy et al., Science 265, (1994), 257-259) oder Fluoreszenzmikroskopie erfolgen.

Alternativ können die erfindungsgemäßen Nukleinsäuren, das Prionprotein oder beide mit Fluoreszenzfarbstoffen markiert sind und die der Wechselwirkung zwischen der Nukleinsäure und dem PrP zu einer meßbaren Veränderung des Fluoreszenzsignals führt. Derartige Veränderungen der Fluoreszenz können auch auf einer Änderung der intrinsischen Fluoreszenz des Prionproteins nach Bindung einer der erfindungsgemäßen Nukleinsäuren beruhen. Ebenfalls zu einer Änderung der intrinsischen Fluoreszenz kann die Veränderung des Prionproteins selbst führen, z.B. durch Einführung einer Trp-Punktmutation, was für das Messignal und dessen Veränderung infolge Bindung der Nukleinsäuren verwendet werden kann. Beispielsweise konnte die Bindung von Thrombin an mit Fluoreszenzfarbstoffen markierten anti-Thrombin-Aptameren durch Messung der Änderung der Fluoreszenz-Anisotropy nachgewiesen werden (Potyrailo R. A. et al., Anal Chem 70 (1998), 3419-25). Dabei konnten bis zu 0,7 amol Thrombin nachgewiesen werden. In einer anderen Ausführungsform ist ein Interaktionspartner, die Nukleinsäure oder das Protein mit einer fluoreszenzlöschenden Gruppe (Quencher), der andere mit einer fluorophoren Gruppe (Donor) markiert ist, wobei die Wechselwirkung zwischen beiden Interaktionspartnern zur Löschung der Fluoreszenzemission des Donors durch den Quencher führt. Findet die Wechselwirkung statt, kann dies durch eine Verminderung des Fluoreszenzsignals gemessen werden. Diese auch als FRET ("fluoreszenz resonanz energy transfer") bezeichnete Methode ist dem Fachmann bekannt und wird auch zur Detektion von Protein/Proteinwechselwirkungen angewendet (Sorkin A. et al., Curr Biol. 10 (2000), 1395-1388; Latif R. und Graves P., Thyroid. 10 (2000), 407-412, 14: Buranda T. et al., Cytometry 37 (1999), 21-31). Dem Fachmann sind die Prinzipien des Nachweises von Interaktionen zwischen Molekülen durch Fluoreszenzmarkierung und die verschiedenen Gestaltungsmöglichkeiten der Nachweise über einfache oder mehrfache Fluoreszenzmarkierung eines oder beider Interaktionspartner vertraut.

Ebenso kann die Detektion der Komplexe aus den erfindungsgemäßen Nukleinsäuren und dem PrP auf geeigneten Sensoren erfolgen, die mit einem der beiden Bindungspartnern beschichtet sind. Die Detektion kann hier über die Zunahme der Masse nach der Bindung des zweiten Bindungspartners erfolgen. Beispielsweise wurden Komplexe aus RNA-Aptameren und dem Enzym 2'-5'-Oligoadenylatzyklase oder RNA-Aptameren und der NS3-Protease des Hepatitis C Virus durch sogenannte SPR-("Surface Plasmon Resonance")-Analysen detektiert (Hartmann et al., J Biol Chem 273 (1998), 3236-3246; Hwang et al., Biochem Biophys Res Commun 279 (2000), 557-562). Andere geeignete Analysemethoden, wie Quarzkristall-Mikrowaagen ("quartz crystal microbalance", QCM) (Kosslinger et al., Biosens Bioelectron 7 (1992), 397-404; Hengerer et al., Biosens Bioelectron 14 (1999), 139-144), sind dem Fachmann ebenfalls bekannt.

In einer besonderen Ausführung kann die Interaktion zwischen dem Protein und dem Nukleinsäureliganden, insbesondere einem Aptazym, durch ein katalytisches Ereignis nachgewiesen werden. Dabei ist die PrP-erkennende Nukleinsäuredomäne mit einer Ribozymdomäne in einer Weise verknüpft, dass die Bindung des PrP's die Aktivität der Ribozymdomäne allosterisch beeinflusst, d.h. aktiviert oder inhibiert. Die Konstruktion von signalgebenden Aptazymen ist dem Fachmann bekannt und in der Literatur beschrieben (Hesselberth et al., J Biotechnol 74 (2000), 15-25; Marshal und Ellington, Nat Struct Biol 6 (1999), 992-994; Soukup und Breaker, Trends Biotechnol 17 (1999), 469-476). Beispiele für Ribozymdomänen, die in Aptazymkonstrukten eingesetzt worden sind, sind effektoraktivierte Ribozymligasen (Robertson und Ellington Nucleic Acids Res 28 (2000),:1751-1759) oder Hammerheadribozyme (Soukup et al., J Mol Biol 298 (2000), 623-632). Der Nachweis der aktiven Ribozymdomäne kann im Fall der Ribozymligasen, die sich selbst mit einem Oligonukleotid verknüpfen über reverse Transcription PCR mit einem zu dem verknüpften Oligonukleotid komplementären Primer erfolgen (Robertson und Ellington, Nat Biotechnol 17 (1999), 62-66). Aktive Hammerheaddomänen können durch Spaltung eines Substratoligonukleotids beispielsweise in einem FRET-Assay nachgewiesen werden (Jenne et al., Angew. Chem. 111 (1999), 1383-1386). Dabei ist das zu spaltende Oligonukleotid mit einer fluoreszenzgebenden (Donor) und einer fluoreszenzlöschenden Gruppe (Quencher) markiert. Im ungespaltenen Zustand sitzen Donor und Quencher in unmittelbarer Nachbarschaft und das vom Donor emitierte Licht wird vom Quencher absorbiert. Nach der Spaltung wird der Donor freigesetzt und die jetzt von ihm frei emittierte Fluoreszenz kann detektiert werden.

Ebenso können die erfindungsgemäßen Nukleinsäureliganden wie Antikörper in dem Fachmann bekannten ELISA-Applikationen eingesetzt werden. Ein Beispiel für die Anwendung eines Aptamers in einer ELISA-Applikation ist der spezifische Nachweis des Proteins VEGF mittels anti-VEGF-Aptameren (Drolet et al., Nat Biotechnol 14 (1996), 1021-1024; Kato et al., Analyst 125 (2000), 1371-1373). Die erfindungsgemäßen Nukleinsäuren können auch zur Detektion der Prionproteine auf Chips als Trägermaterialien eingesetzt werden. Die Anwendung von Nukleinsäureliganden in derartigen Formaten wurde beispielsweise beschrieben in Brody et al. (Mol Diagn 4 (1999), 381-388). Als Probenmaterialien eignen sich dabei unter anderem Körperflüssigkeiten wie Blut, Urin, Lymphflüssigkeit, Vaginalflüssigkeit, Cerebrospinalflüssigkeit, Punktate, Stuhl, Biopsien, zelluläre Proben und Aufschlüsse davon. Daraus ergibt sich der spezifische Nachweis von PrP in den zu untersuchenden Proben

Die Verwendung der erfindungsgemäßen Nukleinsäuren zu diesen Zwecken kann auch mittels sogenannter Kits erfolgen. Im vorliegenden Fall umfasst ein derartiger Kit zumindest eine der erfindungsgemäßen Nukleinsäuren, die optional bereits mit einer der oben beschriebenen Markierungen versehen sind. Weitere Ausführungsformen umfassen weitere Reagenzien, wie z.B. Positiv- und Negativkontrollen. Negativkontrollen können dabei so ausgebildet sein, dass eine Nukleinsäure im Kit enthalten ist, die an PrP, oder bestimmten Formen davon, wie sie hierin auch beschrieben sind, nicht binden oder deren Funktion, insbesondere die PrPsc-Akkumulation, nicht modulieren, und insbesondere diese nicht inhibieren. Als Positivkontrolle kann in einer weiteren Ausführungsform ein PrP beigefügt werden, an den die in dem Kit ebenfalls vorhandene erfindungsgemäße Nukleinsäure bindet. Sowohl die erfindungsgemäße Nukleinsäure als auch das PrP kann in einer Ausführungsform des Kits dabei entweder einzeln oder gemeinsam in einer Zelle, bevorzugterweise exprimiert oder in einer exprimierbaren Form vorliegen. In weiteren Ausführungsformen kann der Kit weiterhin umfassen Puffer, Waschlösungen und dergleichen.

Die Verwendung der erfindungsgemäßen Nukleinsäuren, um einen Komplex mit den hierin beschriebenen Prionproteinen auszubilden, kann neben dem therapeutischen und diagnostischen Bereich auch im technischen oder präparativen Bereich erfolgen. Dabei können die erfindungsgemäßen Nukleinsäuren an Trägermaterialien wie z.B. Sepharosen, Agarosen, oder magnetische Partikel gekoppelt werden. Diese Affinitätsträgermaterialien können daraufhin zur präparativen Aufreinigung der Prionproteine verwendet werden. Entsprechende Protokolle wie Affinitätspräzipitationen, analog zu Immunpräzipitationen, oder Affinitätschromatographien sind dem Fachmann bekannt. Protokolle für analoge Immunpräzipitationen sind beispielsweise beschrieben in Ausubel et al. (Current Protocolls in Molecular Biology (1991), Wiley Interscience , New York) oder Sambrook et al. (Molecular cloning - a laboratory manual, 2^{nd} Ed. (1989), Cold Sprin Harbor Laboratory Press, Cold Spring Harbor). Nach Auftragen der Probe und Bindung des Wechselwirkungspartners kann das Material mit geeigneten Waschlösungen von unspezifischen Bindungspartnern befreit werden. Anschließend wird der spezifisch gebundene Wechselwirkungspartner, d.h. das Prionprotein, eluiert und optional quantifiziert. Als Elutionsmittel bzw. Elutionsbedingungen werden dabei typischerweise hohe Salzkonzentrationen, Detergenzien wie SDS, chaotrope Agenzien wie Harnstoff oder Guanadiniumhydrochlorid verwendet. Desweiteren kann das Protein durch Hitzedenaturierung der Nukleinsäureliganden, typischerweise zwischen 60°C und 95°C oder Denaturierung der Struktur der Nukleinsäureliganden durch Komplexierung zweiwertiger Ionen beispielsweise durch EDTA eluiert werden. Dem Fachmann sind Protokolle, die zur Auflösung der Interaktion zwischen den Nukleinsäureliganden und dem aufzureinigenden Zielprotein verwendet werden können, bekannt. Als Probe können die vorstehend genannten Proben verwendet werden. Zusätzlich kann infolge des präparativen Charakters dieser Ausführungsform die Probe auch eine Kultivierungsmedium für Zellen sein, die den jeweiligen Bindungspartner bilden, mit oder ohne Zellen (im Falle des Exports des Bindungspartners), bzw. ein Aufschluß von Zellen sein. Ein Protokoll für die Aufreinigung von in CHO-Zellen exprimiertem humanem L-Selektin mittels eines spezifischen Aptamers über Affinitätschromatographie wurde von Romig und Mitarbeitern beschrieben (Romig, J Chromatogr B Biomed Sci Appl 731 (1999), 275-284)

### Beispiele und Figuren

Die Erfindung wird im folgenden anhand der Beispiele und Figuren erläutert, aus denen sich weitere Merkmale, Ausführungsformen und Vorteile der verschiedenen Aspekte der Erfindung ergeben. Dabei zeigt:
- Fig. 1: einen schematischen Aufbau der Primärstruktur des Prionproteins des Hamsters;
- Fig. 2: die NMR-Struktur des humanen PrP^{C} im Bereich der Aminosäuren 23 bis 230;
- Fig. 3: Modelle zur Prionproteinpropagation;
- Fig. 4: eine schematische Darstellung des Heterodimer-Modells (A) sowie des kernabhängigen Polymerisations-Modelles (B);
- Fig. 5: den Aufbau des Selektionstargets;
- Fig. 6: den Verlauf der Selektion von Prionpeptid-spezifischen Aptameren bis zum achten Selektionszyklus;
- Fig. 7: den Verlauf der Prionpeptid-Selektion ab dem achten Selektionszyklus;
- Fig. 8: eine Übersicht der zu Sequenzfamilien I bis III gruppierten erfindungsgemäßen Aptamern sowie des als "orpham" bezeichneten einzelnen Klons, der ebenfalls ein erfindungsgemäßes Aptamer darstellt,
- Fig. 9: die relative Bindung verschiedener Sequenzmotive und dessen unselektierten Pools am Prionpeptid AS 90 bis 129;
- Fig. 10: eine schematische Darstellung des Experiments, bei dem der Wirkmechanismus eines der erfindungsgemäßen Aptamere bestimmt wurde;
- Fig. 11: das Ergebnis einer Analyse des de novo synthetisierten PrP^{Sc} aus peristent Prioninfizierten Zellen;
- Fig. 12: den relativen Anteil an Proteinase K-resistenten PrP^{Sc};
- Fig. 13: ein modifiziertes Modell zur Prionprotein-Entstehung;
- Fig. 14: den schematischen Aufbau der kombinatorischen DNA- und RNA-Bibliothek des Mic Mod 40 N-Pools;
- Fig. 15: eine schematische Darstellung eines Aptazyms und
- Fig. 16: ein spezifisches Pol III-Expressionssystem.

Bei dem in Fig. 1 gezeigten schematischen Aufbau der Pimärstruktur des Prionproteins des Hamsters können den verschiedenen Bereichen die folgenden Funktionen bzw. Merkmale zugewiesen werden. Die folgenden Zahlen geben die Aminosäurereste an:: 1-22 Signalpeptid, 232-254 Signalsequenz, OR= Oktarepeatregion, pK= Proteinase K. Die Asparaginreste (CHO) N181 und N197 repräsentieren Glykosilierungsstellen. Die Disulfidbrücke (S-S) befindet sich zwischen den Cysteinresten C179 und C214. Die pK-sensitive Region erstreckt sich bis AS 90, pK-Behandlung führt zur PrP^{C}-Degradation und N-terminalen Verkürzung von PrP^{Sc} zur 27-30 kDa Form.

Fig. 2 zeigt die NMR Struktur des humanen PrP^{C} im Bereich der Aminosäuren 23-230. Die beiden caboxyterminalen α Helices α₂ AS 173-194 und α₃ AS 200-228 sind V-förmig gegeneinander verdreht und durch eine Disufidbrücke zwischen der AS C179 und C214 verbunden. Die aminoterminale α₁ Helix AS 144-154 ist von zwei antiparallelen β-Faltblätteren flankiert (β₁ AS 128-131, β₂ AS 161-164) (Quelle [Zahn et al., Proc Natl Acad Sci U S A 97 (2000), 145-50]).

Fig. 3 zeigt die derzeit diskutierten Modelle zur Prionpropagation, die nach Weissmann modifiziert sind [Weissmann, Prog Brain Res 105 (1995), 15-22]:
A) nicht infizierte, normale Zelle: PrP^{C} wird synthetisiert, zur Zelloberfläche transportiert und reinternalisiert
B) Das Protein-only-Modell: Das zelluläre PrP wird exogen durch PrP^{Sc} bzw. endogen durch Mutationen oder sporadisch in PrP^{SC} konvertiert. Die Konversion findet entweder an der Zelloberfläche oder nach Internalisierung von PrP^{Sc} statt.
C) Das Virino-Modell: Das infektiöse Agens, eine Nukleinsäure, umhüllt von PrP^{Sc}, gelangt vermutlich über eine Rezeptor-vermittelte Endozytose in die Zelle und repliziert sich dort mit Hilfe der zellulären Replikationsmaschinerie. Die Tochtermoleküle bilden mit PrP^{C} neue Virionen.

Fig.4 zeigt in A) das Heterodimermodell und in B) das Kernabhängige Polymerisationsmodell

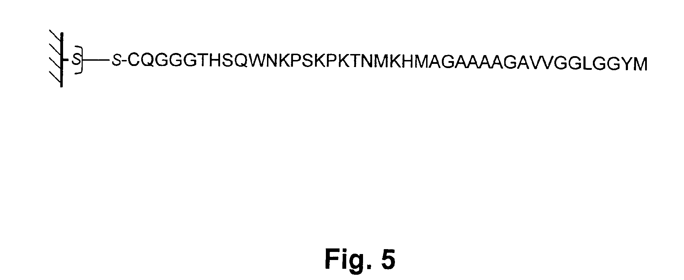

Fig. 5 zeigt das erfindungsgemäße Selektionstarget: Humanes Prionpeptid AS 90-129, welches über einen zusätzlichen N-terminalen Cysteinrest an Thiolsepharose 4B gekoppelt wurde. Prionpeptid AS 90 -- 129 bedeutet dabei, dass das Peptid eine Aminosäureregion umfasst, die den Aminosäurepositionen 90 bis 129 des humanen Prionproteins entspricht. Sofern hierin Bezug genommen und nichts Gegenteiliges vermerkt ist, handelt es sich bei dem Begriff des Prionpeptids um das Prionpeptid AS 90 - 129.

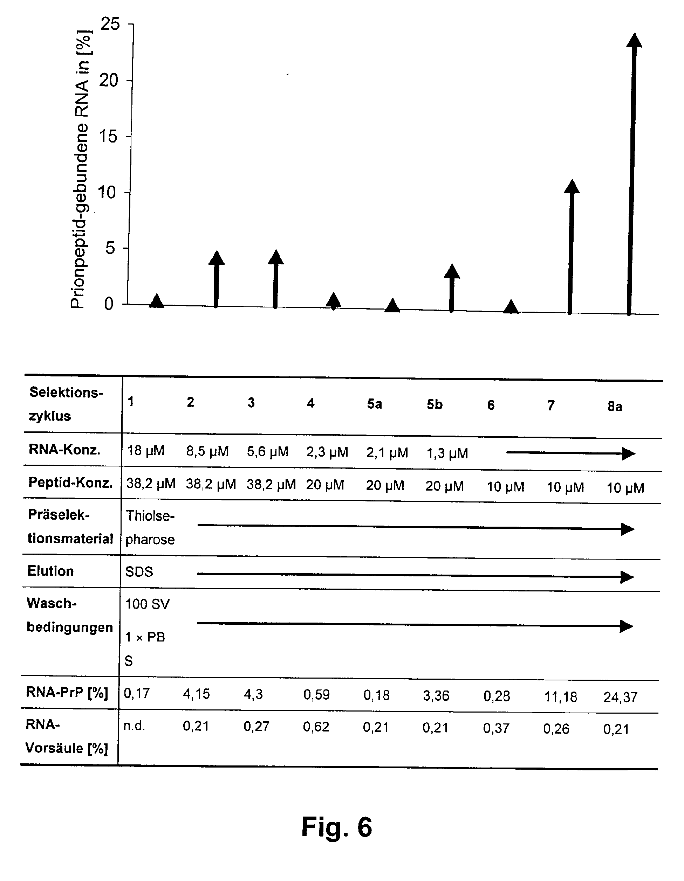

Fig. 6 zeigt den Verlauf der Selektion von Prionpeptid-spezifischen Aptameren bis zum achten Zyklus. Dabei ist der relative Anteil an spezifisch gebundener RNA in [%] für die einzelnen Selektionszyklen aufgetragen (Abzisse). Die Selektions- und Präselektionsbedingungen sowie die Ergebnisse der Selektion und Präselektion sind in Tabelle 3 wiedergegeben. Das Eluat von Zyklus 5a war Ausgangspunkt für die weiteren Selektionsschritte. (n.d. = nicht durchgeführt, SV = Säulenvolumen)

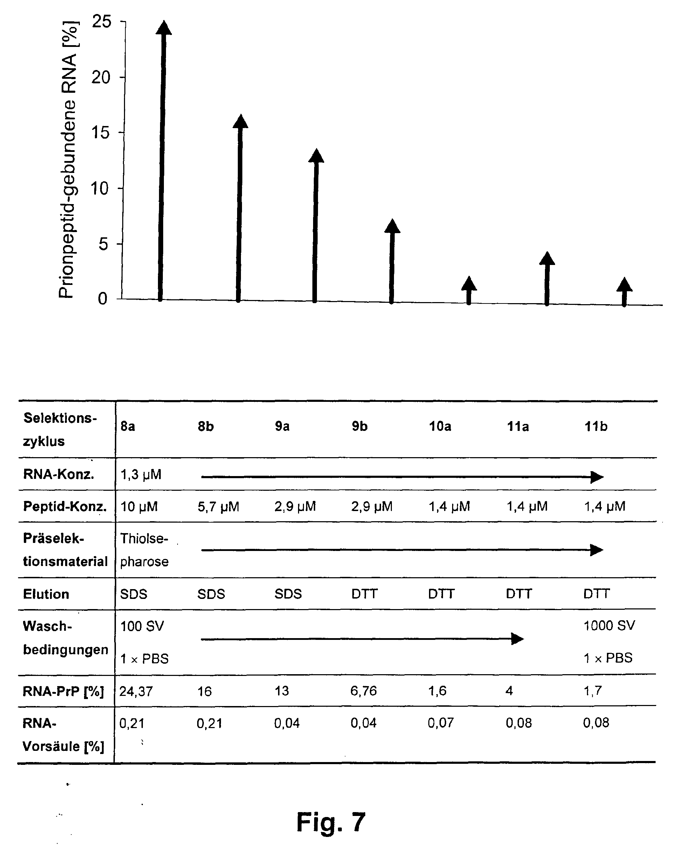

Fig. 7 zeigt den Verlauf der Prionpeptid-Selektion ab dem achten Selektionszyklus: Der relative Anteil an spezifisch gebundener RNA in [%] ist für die einzelnen Selektionszyklen aufgetragen (Abzisse). Die Selektions- und Präselektionsbedingungen sowie die Ergebnisse der Selektion und Präselektion sind in Tabelle 4 zusammengefaßt (SV=Säulenvolumen).

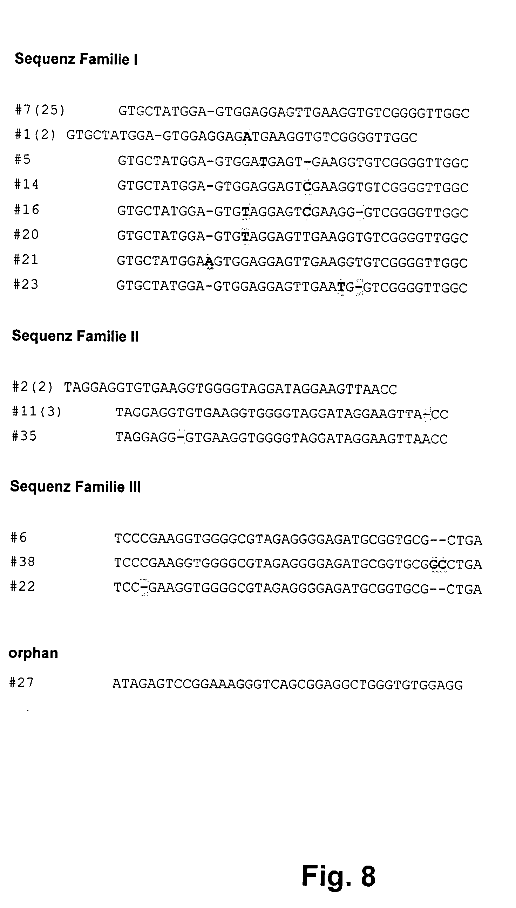

Fig. 8 ist eine Übersicht der Sequenzen der Klone aus der Prionpeptid-Selektion (ohne Primerbindungsstellen), wobei für jeden Klon nur die urspünglich randomisierte Region 40N gezeigt ist. Die Anzahl mehrfach isolierter Sequenzen unter den 43 analysierten Klonen ist in Klammern angegeben. Die 2'-Amino-2'-deoxycytidin- and 2'-Amino-2'-deoxyuridine-Reste sind als C oder T bezeichnet. Mutationen und Deletionen (-) innerhalb einer Familie im Vergleich zur ersten dargestellten Sequenz sind grau unterlegt.

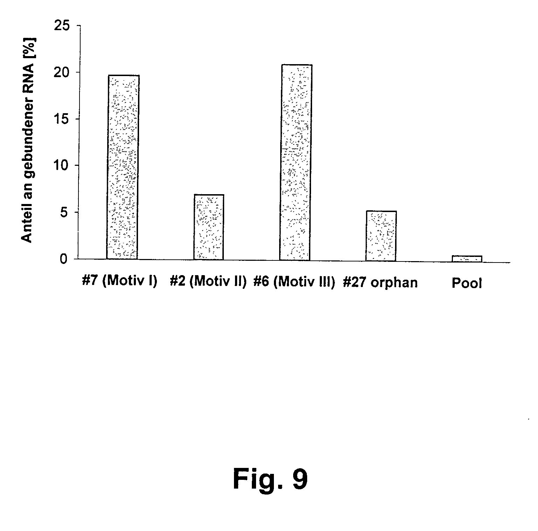

Fig. 9 ist eine Darstellung der relativen Bindung verschiedener Sequenzmotive und des unselektierten Pools am Prionpeptid AS 90-129. Die Bindungsreaktion wurde mit immobilisiertem Prionpeptid (400 pmol), radioaktiv markierter RNA (200 pmol) in PBS Bindungspuffer (ph 7,4) und 1 µg/µl Heparin durchgeführt. Auf der Ordinate ist der relative Anteil an peptidgebundener RNA zur eingesetzten Gesamtradioaktivität (100%) dargestellt.

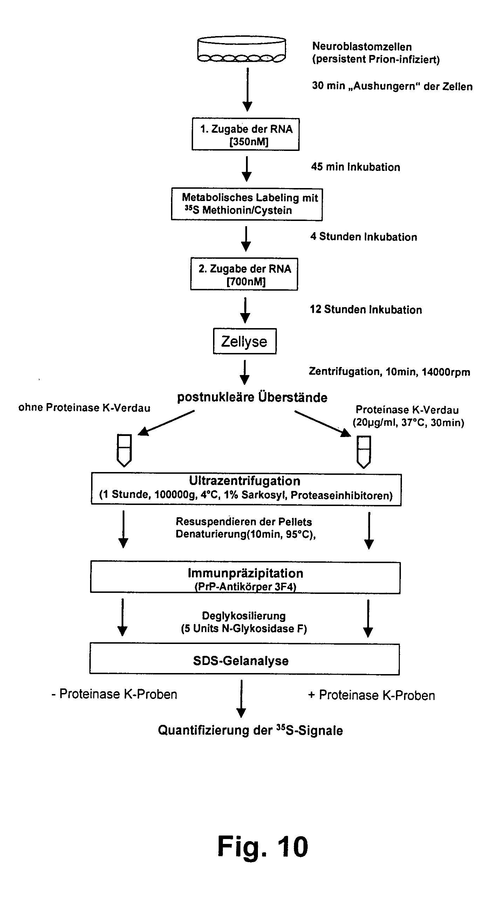

Fig. 10 ist eine schematische Darstellung des Experimentes, bei dem der Wirkmechanismus eines der erfindungsgemäßen Aptamere bestimmt wurde.

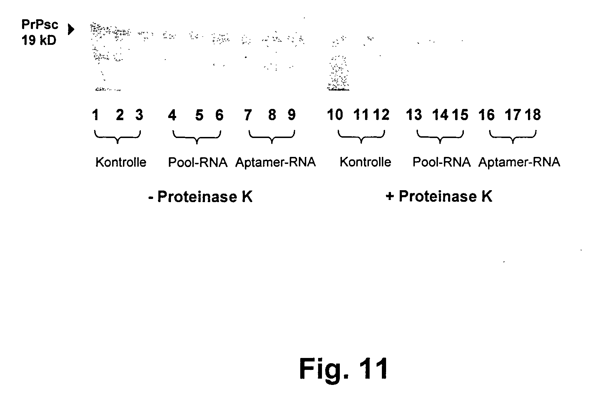

Fig. 11 zeigt das Ergebnis einer Analyse des de novo synthetisierten PrP^{Sc} aus persistent Prion infizierten Zellen:
Spur 1-9 unlösliches PrP^{Sc}, Spur 10-18 unlösliches, pK-resistentes PrP^{Sc} Spur 1-3 und 10-12 Kontrollansatz ohne RNA, Spur 4-6 und Spur 13-15 Behandlung mit Pool-RNA, Spur 7-9 und Spur 16-18 Behandlung mit #7 Aptamer. Die Proben wurden auf ein 12,5%iges PAA Gel aufgetragen und das ³⁵S-Signal im Phosphorimager quantifiziert

Fig. 12 ist ein Säulendiagramm, das den relativen Anteil an ProteinaseK-resistenten PrP^{Sc} bezogen auf die Gesamtmenge des de novo synthetisiert PrP^{Sc} darstellt. Die Prion infizierten N₂a-Zellen wurden mit 0,7 µM Aptamer-RNA (Motiv I), 0,7 µM Pool-RNA bzw. PBS-Puffer inkubiert.

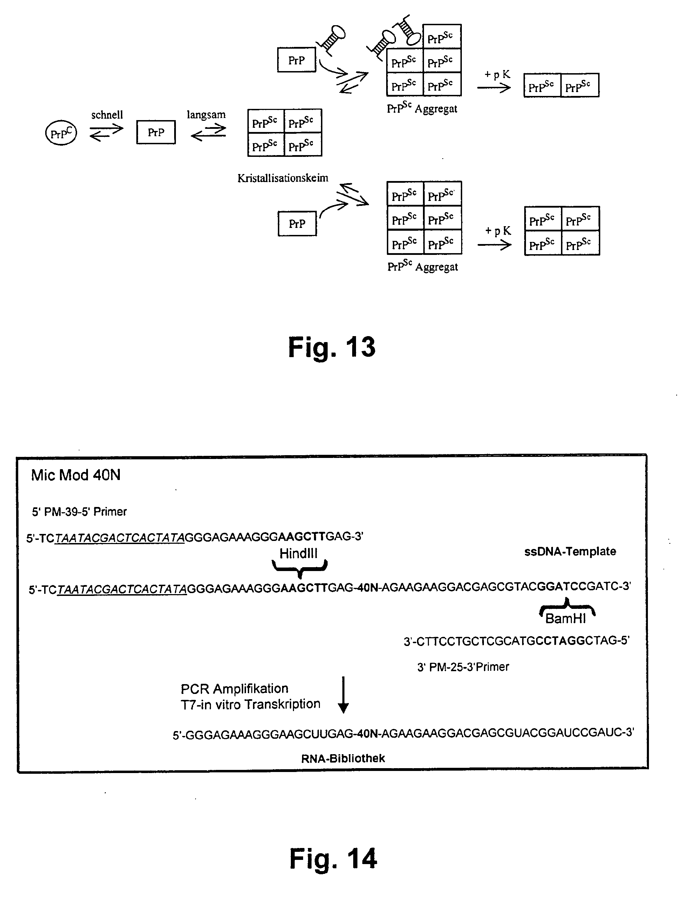

Fig. 13 zeigt ein modifiziertes Modell zur Prionproteinentstehung nach Caughey: Die Ausbildung von hochmolekularen PrP^{Sc}-Aggregaten aus dem Kristallisationskeim wird in Gegenwart der Aptamere beeinflußt. Mit der Aggregatbildung verbunden ist die Akquisition der ProteinaseK-Resistenz. In Gegenwart der Aptamere kommt es zur Reduktion der pK-Resistenz als Maß für eine veränderte oder verminderte Aggregatbildung (Caughey et al. 1993).

Fig. 14 zeigt den Aufbau der kombinatorischen DNA- und RNA-Bibliothek des Mic Mod 40N-Pools. Nach PCR-Amplifikalion der ss-DNA-Templates mittels des 5'-Primer's PM-39-5' und des 3'-Primer's PM-25-3' ist die erhaltene ds DNA Ausgangspunkt für die in vitro Transkription der RNA-Bibliothek, kursiv und unterstrichen: T7-RNA-Polymerase-Promotor zur Transkription der RNA Bibliotheken, fett: Restriktionsschnittstellen, N40 stellt den randomisierten Bereich von 40 Basen dar, wobei alle Basen im gleichen Verhältnis zueinander in der Synthese des ss-DNA-Templates vorlagen. Der N40-Bereich ist flankiert von konstanten Sequenzabschnitten, die die Primerbindestellen für die enzymatische Amplifikation in der reversen Transkriptionsreaktion und PCR-Reaktion darstellen.

### Beispiel 1: In vitro Selektion von RNA-Molekülen an einer ausgewählten Region des humanen Prionproteins

### Charakterisierung der verwendeten Nukleinsäurebibliothek

Für die Selektion am Prionpeptid AS90-129 wurde der Pool bzw. die Nukleinsäurebibliothek Mic Mod 40N verwendet. Durch die spezielle Konstruktion des Pools war es sowohl möglich, Selektionen mit 2'-Hydroxy-Ribonukleinsäuren als auch mit 2'-modifizierten, wie beispielsweise 2'-Amino- oder 2'-Fluoro)-Ribonukleinsäuren, genauer 2'-Amino-2'Deoxy-modifizierten Pyrimidinen, welche in der *in vitro* Transkription in die Ribonukleinsäuren eingebaut wurden, durchzuführen. Diese Modifikation der RNA erhöht deren Stabilität gegen die Mehrheit der im Serum vorkommenden Ribonukleasen [Jellinek et al., Biochemistry 34 (1995), 11363-72], eine Voraussetzung für die funktionelle Charakterisierung der Aptamere in biologischen Systemen. Für eine effektive Transkription der Template mit 2' modifizierten Nukleotiden wurden beispielsweise Pyrimidinbasen innerhalb der ersten 12 Positionen vermieden (vgl. Fig. 14) Ebenso enthalten die RNA-Moleküle an der Initationsstelle der reversen Transkription in ihren 3'-konstanten Bereichen Purine, um die Polymerasereaktion des Enzyms zu fördern [Blind, Doktorarbeit (2000)]

Die DNA-Bibliothek-Mic Mod 40N enthält einen randomisierten Bereich von 40 Nukleotiden, welcher am 3' und 5' Ende von invarianten Primerbindestellen für die nach jedem Selektionszyklus durchgeführte reverse Transkription und PCR-Amplifikation flankiert ist. Über den 5'-Primer wird zudem der für die *in vitro* Transkription erforderliche T7-Promotor eingeführt. Die Komplexität der Nukleinsäurebibliothek beträgt ca. 8,8 × 10¹⁵ verschiedene Moleküle [Blind, Doktorarbeit (2000)]. Durch ein affinitätschromatographisches Verfahren konnten die spezifisch bindenden Aptamere an einer mit dem Zielmolekül derivatisierten Sepharosematrix angereichert werden. Durch einen Präselektionschritt mit der doppelten Menge an Vorsäulenmaterial verglichen zur mit Prionpeptid AS 90-129 immobilisierten Sepharose, wurden diejenigen RNA-Moleküle entfernt, die unspezifisch an das Trägermaterial binden.

### Charakterisierung des Selektionstargets

Bei der Selektion von Prionpeptid-Aptameren wurde ein affinitätschromatographisches Verfahren verwendet, bei dem das in Fig. 5 dargestellte, an Thiolsepharose 4B gekoppelte Selektionstarget verwendet wurde.

### Kopplung des Prionpeptids (AS 90-129) an Thiol-Sepharose 4B

Mittels aktivierter Thiolsepharose 4B ist es möglich, Peptide mit freien Thiolgruppen unter Ausbildung einer Disulfidbrücke zu koppeln. Dabei wird 2-Thiopyridone freigesetzt, welches ein Maß für die Kopplungseffizienz darstellt. Die Konzentration des freigesetzten 2-Thiopyridons wird spektroskopisch bei einer Wellenlänge von λ= 343 nm (ε = 7060/Mcm) ermittelt. 500 mg von der Sepharose 4B wurden eingewogen und in 3,5 ml entgastem H₂O aufgenommen. Anschließend wurde die gequollene Sepharose mit 60 ml entgastem H₂O gewaschen, um Stabilisatoren des gefriergetrockneten Pulvers zu entfernen, und 1 h bei 4°C in 3,5 ml Kopplungspuffer äquilibriert. Die Kopplung des N-terminal mit einem Cysteinrest sythetisierten Prionpeptides erfolgte über Nacht bei 4°C im Überkopfschüttler.

Als Kontrolle wurde ein Ansatz mitgeführt, welcher nur Sepharose enthielt und analog zum Peptidansatz behandelt wurde. Nach der Immobilisierung wurde das Sepharosematerial in eine Bio-Rad-Säule überführt und der Durchfluß spektroskopisch vermessen. Die Kopplungseffizienz lag zwischen 45 %-60 %. Bevor die verbleibenden 2-Thiopyridyl-Reste durch die Inkubation des Ansatzes für 30 min bei 4°C mit 5 mM Mercaptoethanol in NaOAc (100 mM; pH 6,0) beseitigt wurden, erfolgte ein Waschschritt mit 7 ml der 100 mM NaOAc-Lösung. Das immobilisierte Sepharosematerial konnte im Bindungspuffer maximal 14 Tage bei 4°C aufbewahrt werden.

| Kopplungspuffer: | | Bindungspuffer: |
|---|---|---|
| NaCI | 500 mM | 1 × PBS |
| EDTA | 1 mM | Triton X100 0,1 % |
| Tris/HCL, pH 8,4 | 10 mM | |

### Durchführung der Selektion

Der Ablauf dieser Selektion ist in den Fig. 6 und 7 zusammengefaßt. Im ersten Zyklus wurden 8,4 Genomkopien des Pools Mic Mod 40N eingesetzt. In den folgenden Zyklen wurde die doppelte Menge an Sepharosematerial im Vergleich zum derivatisierten Material verwendet. Die gleichbleibend niedrigen Werte in der Präselektion zeigen, dass keine unspezifischen Vorsäulenbinder angereichert wurden (vgl. Tab. 3, Tab. 4). Durch die Verwendung von 0,5%igem SDS-Puffer in den Zyklen 1-9 a gelang eine vollständige Elution der gebundenen Aptamere. Die resultierende cDNA nach Zyklus 5a war Ausgangspunkt für die folgenden Selektionszyklen. Ein deutlicher Anstieg des Anteils an peptidgebundener RNA auf 11 % war im Zyklus 7 zu beobachten. Die Zunahme an affinen RNA-Molekülen setzte sich in der achten Selektionsrunde fort, in der 24 % der auf die derivatisierte Säule gegebenen RNA-Moleküle eluiert werden konnten.

Um besonders affine PrP-Binder aus der bereits angereicherten RNA-Bibliothek zu selektieren, wurde die Stringenz der Selektion in den folgenden Zyklen erhöht. Dies wurde durch die Erniedrigung des Derivatisierungsgrades der Prionpeptidsäule im Vergleich zum vorangegangenen Zyklus (Zyklus 8b, 9 und 10) und die Erhöhung der Waschvolumina (Zyklus 11) erreicht. Bedingt durch den zusätzlichen Selektionsdruck reduzierte sich der Anteil an gebundener RNA (vgl. Fig. 7, Tab. 4). In den Zyklen 9,10 und 11 wurden nur die affinsten Binder, welche in 8b, 9b bzw. 11b eluiert worden waren, weiter verwendet. Da durch die unspezifische Elution mit SDS-Puffer eventuell bifunktionelle Aptamere, die das Prionpeptid nur im Komplex mit dem Matrixmaterial erkennen, angereichert werden könnten, ging man ab Zyklus 9b zur spezifischen Elution durch Reduktion der Disulfidbrücke zwischen den Thiolgruppen der Matrix und dem N-terminalen Cysteinrest des Peptides mittels 500mM DTT über. Dabei wird deutlich (vgl.Fig. 7, Tab. 4, Zyklus:9a und 9b), daß durch diesen Elutionsschritt nur etwa 50% der gebundenen RNA im Vergleich zur unspezifischen Elution erhalten werden. Ein möglicher Grund für die Verringerung der Elutionsausbeute mit DTT ist die Maskierung und damit Unzugänglichkeit der Disulfidbrücke für DTT durch Ausbildung von Sekundärstrukturen des Peptides oder Konformationsänderungen des RNA/Peptid-Komplexes.

Nach 12 Zyklen wurde die Peptid-gebundene RNA eluiert, revers transkribiert, PCR-amplifiziert und die erhaltene ds DNA in den Vektor pGEM 4Z kloniert und 43 Klone sequenziert. Die Primärstruktur der erhaltenen Sequenzen erlaubt eine Einteilung in drei Sequenzfamilien, wie sie auch in Fig. 8 dargestellt ist. Die Basenabfolge von Klon 7 der Familie I ist mit 25 identischen Sequenzen die am häufigsten vorkommende, hoch konservierte Sequenz der Prionpeptidselektion. Die Familie I repräsentiert mit 77 % den größten Anteil der erhaltenen individuellen RNA-Moleküle, während Familie II 14% ausmacht und Familie III etwa 7%. Neben den in Abbildung 32 gezeigten Sequenzfamilien wurde noch ein Klon (#27) selektiert, welcher keiner der Familien zuzuordnen ist. Die Mitglieder einer Familie unterscheiden sich nur durch Punktmutationen, Deletionen und Insertionen. Unter den drei Sequenzfamilien waren keine Sequenzhomologien feststellbar. Aus jeder Familie wurden repräsentative Sequenzen für weitere Charakterisierungen herangezogen.

Auf der Grundlage der erhaltenen drei Sequenzfamilien wurden die folgenden Konsensus-Sequenzen ermittelt.

Konsensus-Sequenz der Sequenz-Familie I, die hierin als SEQ.ID.No. 1 bezeichnet wird: KKG MTR TGG ARG WGD WKR AGK YGW MKG KGT YGG S wobei an Position 1 K entweder G oder T,
Position 2 K entweder G oder T,
Position 4 M entweder A oder C,
Position 5 entweder T oder unbesetzt,
Position 6 R entweder G oder A,
Position 11 R entweder G oder A oder unbesetzt,
Position 13 W entweder A oder T,
Position 15 D entweder A oder G oder T,
Position 16 W entweder A oder T
Position 17 K entweder G der T,
Position 18 R entweder A oder G,
Position 21 K entweder G oder T,
Position 22 Y entweder T oder C oder unbesetzt,
Position 24 W entweder A oder T,
Position 25 M entweder A oder C,
Position 26 K entweder G oder C,
Position 28 K entweder G oder T oder unbesetzt,
Position 31 Y entweder T oder C und
Position 34 S entweder G oder C ist.

Konsensus-Sequenz der Sequenz-Familie II, die hierin als SEQ.ID.No. 2 bezeichnet wird: KWGKR RRKGT GRRGK WGGRK WRGRA KWKRA MS wobei an Position 1 K entweder G oder T,
Position 2 W entweder A oder T,
Position 4 K entweder G oder T,
Position 5 R entweder A oder G,
Position 6 R entweder A oder G,
Position 7 R entweder A oder G,
Position 8 K entweder G oder T,
Position 12 R entweder A oder G,
Position 13 R entweder A oder G
Position 15 K entweder G oder T
Position 16 W entweder A oder T,
Position 19 R entweder A oder G
Position 20 K entweder G oder T
Position 21 W entweder A oder T
Position 22 R entweder A oder G
Position 24 R entweder A oder G
Position 26 K entweder G oder T
Position 27 W entweder A oder T
Position 28 K entweder G oder T,
Position 29 R entweder A oder G
Position 31 M entweder A oder C und
Position 32 S entweder G oder C ist.

Konsensus-Sequenz der Sequenz-Familie III, die hierin als SEQ.ID.No. 3 bezeichnet wird: TCCCG AAGGT GGGGC GTAGA GGGGA GATGC GGTGC GGCCT GA wobei an Position 4 C C oder unbesetzt,
Position 37 G G oder unbesetzt, und
Position 38 C C oder unbesetzt ist.

Den verschiedenen zu den einzelnen Sequenz-Familien zu gehörigen Sequenzen, wie sie auch in Fig. 8 dargestellt sind, werden hierin auch wie folgt bezeichnet:

Sequenz Familie I

#7(25)    GTGCTATGGA-GTGGAGGAGTTGAAGGTGTCGGGGTTGGC    : SEQ.ID.No. 4
#1(2)    GTGCTATGGA-GTGGAGGAGATGAAGGTGTCGGGGTTGGC    : SEQ.ID.No. 5
#5    GTGCTATGGA-GTGGATGAGT-GAAGGTGTCGGGGTTGGC    : SEQ.ID.No. 6

#14 GTGCTATGGA-GTGGAGGAGTCGAAGGTGTCGGGGTTGGC : SEQ.ID.No. 7
#16 GTGCTATGGA-GTGTAGGAGTCGAAGG-GTCGGGGTTGGC : SEQ.ID.No. 8
#20 GTGCTATGGA-GTGTAGGAGTTGAAGGTGTCGGGGTTGGC : SEQ.ID.No. 9
#21 GTGCTATGGAAGTGGAGGAGTTGAAGGTGTCGGGGTTGGC : SEQ.ID.No. 10
#23 GTGCTATGGA-GTGGAGGAGTTGAATG-GTCGGGGTTGGC : SEQ.ID.No. 11

Sequenz Familie II

#2(2)    TAGGAGGTGTGAAGGTGGGGTAGGATAGGAAGTTAACC    : SEQ.ID.No. 12
#11(3)    TAGGAGGTGTGAAGGTGGGGTAGGATAGGAAGTTA-CC    : SEQ.ID.No. 13
#35    TAGGAGG-GTGAAGGTGGGGTAGGATAGGAAGTTAACC    : SEQ.ID.No. 14

Sequenz Familie III

#6 TCCCGAAGGTGGGGCGTAGAGGGGAGATGCGGTGCG—CTGA : SEQ.ID.No. 15
#38 TCCCGAAGGTGGGGCGTAGAGGGGAGATGCGGTGCGGCCTGA : SEQ.ID.No. 16
#22 TCC-GAAGGTGGGGCGTAGAGGGGAGATGCGGTGCG—CTGA : SEQ.ID.No. 17

orphan

#27 ATAGAGTCCGGAAAGGGTCAGCGGAGGCTGGGTGTGGAGG :SEQ.ID.No. 18

### Beispiel 2: Bestimmung der Dissoziationskonstanten individueller Sequenzen durch Nitrozellulosefilterbindungsstudien am Prionprotein

Bevor man zur Bestimmung der Dissoziationskonstanten repräsentativer Sequenzen aus der Prionpeptidselektion am rekombinanten Prionprotein überging, wurde die Bindungsfähigkeit der ausgewählten Klone und einer zufälligen Sequenz aus dem unselektierten Pools am immobilisierten Prionpeptid getestet. Dabei wurde ein molares Verhältnis von Peptid : RNA von 2:1 eingesetzt. Aus Fig. 9 ist zu erkennen, daß alle untersuchten Klone mit Ausnahme der unselektierten Sequenz das Prionpetid binden können, wobei sich die repräsentativen Sequenzen aus Familie I (#7) und III (#6) im Vergleich zur Familie II (# 2) und der einmalig vorkommenden Sequenz (#27) in ihrem Bindungsverhalten unterscheiden.

Da alle weiteren Untersuchungen zur Charakterisierung der Aptamere in persistent Prion-infizierten ScN₂a-Zellen, die mit einem chimären Maus-Hamster-Maus PrP-Konstrukt stabil transfiziert worden sind, durchgeführt werden sollten, wurde für die Bestimmung der Dissoziationskonstanten der individuellen Sequenzen rekombinantes humanes Prionprotein, rekombinantes Syrian Hamster (SHa) Prion Protein, chimäres Maus-Hamster-Maus-Prion Protein verwendet. Die rekombinanten Prionproteine wurden in steigenden Konzentrationen (10 nM-10 µM) mit radioaktiv markierter RNA (3 nM) im Bindungspuffer in Gegenwart von 1 µg/µl Heparin und 1 mM MgCl₂ inkubiert. Heparin wurde sowohl als unspezifischer Kompetitor als auch zur Verbesserung der Löslichkeit des sehr schnell zur Präzipitation neigendem PrP zugesetzt. Über Nitrozellulosefiltration wurde gebundene von ungebundener RNA separiert und die auf der Filtermembran zurückgehaltene, komplexierte RNA mittels Phosphorimager quantifiziert. Aus den erhaltenen Bindungskinetiken wurden durch Verwendung der Gleichung 1, die in der Tabelle 5 dargestellten Dissoziationskonstanten für die einzelnen Klone berechnet. Für den unselektierten Pool konnte kein Kd-Wert bestimmt werden, da bei einer Proteinkonzentration von 10 µM zwar 8% an unselektierter RNA komplexiert vorlagen, jedoch keine Sättigungskonzentration des Prionproteins erreicht wurde. Im Falle der bindenden Sequenzen wurden bei einer Proteinkonzentration von 10 µM ca. 40-50% (#2, #7) bzw. 30-35% (#6, #27) der eingesetzten RNA gebunden. Die Verwendung von höheren Proteinkonzentrationen zur exakten Kd-Bestimmung, ist aufgrund der Präzipitationsneigung des PrP selbst in Gegenwart verschiedener Detergentien nicht möglich.

Es konnte jedoch in den Filterbindungsstudien gezeigt werden, daß die selektierten Prionpeptid-Aptamere auch in der Lage sind, das gesamte rekombinante Prionprotein in Lösung zu erkennen (vgl. Tab. 5). Entsprechend den Peptidbindungsstudien (vgl. Fig. 9 weisen die Klone aus den Sequenzfamilien I und III die höchste Affinität zum rekombinanten Hamster-PrP auf. Für die Bindungsuntersuchungen an weiteren rekombinanten Prionproteinen (vgl. Tab. 6) und Untersuchungen im Zellkultursystem wurde Aptamer # 7, welches sich durch die beste Affinität und das beste Komplexbildungsverhalten (50%) innerhalb der analysierten Sequenzen auszeichnet, herangezogen.

| | ***Klon*** | **Kd (µM)** |
|---|---|---|
| Motiv I | #7 | 0,17 |
| Motiv II | #2 | 10,1 |
| Motiv III | #6 | 2,7 |
| Orphan | #27 | 6,9 |
| Pool | #11 | n.d. |

Tabelle 5 Dissoziationskonstanten (Kd-Werte) individueller Sequenzen verschiedener Motive im Filterbindungsassay, (n.d. nicht definierbar). Die folgende Gleichung zur Berechnung der Dissoziationskonstanten wurde dabei verwendet: q= (f/2Rₜ)= (Pₜ + Rₜ +Kd-[(Pₜ +Rₜ + Kd)² -4Pₜ Rₜ ]^{1/2} ; wobei q der Anteil an im Gleichgewicht gebundener RNA ist, Pₜ und Rₜ die Gesamt-Peptid/Protein bzw. RNA-Konzentrationen sind und f die Effizienz an auf den Nitrozellulosefiltern zurückgehaltenen RNA-Peptid/Protein-Komplexen darstellt (Jellinek et al., 1994).

**Tabelle 6**

| In Filterbindungsstudien ermittelte Dissoziationskonstanten (kd-Werte) für die Bindung rekombinanter Prionproteine unterschiedlicher Spezies an das Aptamer Motiv I (n.d. nicht definierbar). | | |
|---|---|---|
| *Prion -Protein* | *Aptamer Motiv I (# 7)* | *unselektierte Pool-RNA* |
| Hamster | 171 nM | n. d. |
| Maus | 100 nM | n. d. |
| Mensch | 1700 nM | n. d. |
| Maus-Hamster-Maus | 199 nM | n. d. |

### Beispiel 3: Einfluß der Aptamere auf die Biogenese und Akkumulation von hochmolekularen PrP^{Sc}-Molekülen in prion-infizierten Neuroblastomzellen

Im folgenden sollte in einem Modellsystem, den persistent Prion infizierten ScN₂a-Zellen, untersucht werden, ob das Motiv I Aptamer (#7) nach Zugabe in das Medium die Neuentstehung von PrP^{Sc} oder die Akkumulation des neuentstandenen PrP^{Sc} in Form von hochmolekularen Aggregaten im Zellkutursystem beeinflußt. In Fig. 10 ist der Ablauf dieses Experimentes schematisch dargestellt.

Das detaillierte Versuchsprotokoll kann dabei wie folgt zusammengefasst werden:

Die Zellen wurden 1-2 Tage vor Verwendung passagiert und in 6 cm Petrischalen ausgesät. Nach 1-2 Tagen waren sie bis zu einer 70%igen Konfluenz gewachsen, der Zellüberstand wurden in 1N NaOH entsorgt und die Zellen wurden in Methionin- und Cystein-freiem RPMI Medium (1 %FKS) im Brutschrank ausgehungert. Nach 30 min erfolgte die Zugabe von im Bindungspuffer de-und renaturierter RNA, wobei im Medium eine Konzentration von 0,35 µM eingestellt wurde. Die unselektierte Pool-RNA und ein Ansatz ohne RNA-Zusatz wurde als Kontrolle mitgeführt. Da Zellsysteme von hoher Komplexität sind und die Reproduzierbarkeit der Ergebnisse gewährleistet werden muß, wurden für jeden Ansatz mindestens Triplettbestimmungen (jeweils drei 6 cm Schalen für die Aptamer-RNA, die Pool-RNA und den Kontrollansatz ohne RNA) vorgenommen. Nach 45 min Inkubation wurde den Zellschalen 800 µCi ³⁵S Methionin/Cystein und dialysiertes FKS (final 3 %) zugesetzt, um die Translationsmaschinerie nicht weiter zu blockieren. Eine zweite Zugabe der RNA erfolgte nach 4 Stunden, so daß am Ende eine Konzentration von 0,7 µM RNA im Medium vorlag (vgl. Fig. 10).

Nach 12 Stunden wurden die Zellen lysiert, kurz abzentrifugiert und die postnukleären Überstande zu jeweils 500 µl aufgeteilt. Ein Teil wurde 30 min bei 37°C einen Proteinase K-Verdau (20µg/ml) unterzogen. Vor dem Ultrazentrifugationsschritt setzte man Proteaseinhibitoren 10 µl Aprotinin (SL 14 mg in 5 ml EtOH), 10 µl Pefabloc (SL 50 mM in Wasser und 50 µl Sarkosyl (SL 10 %) hinzu und zentrifugierte 1 h bei 100.000g und 4°C im TLA 45 Rotor. Die Pellets wurden in 100 µl Waschpuffer/SDS resuspendiert, 10 min bei 95°C aufgekocht, kurz auf Eis gestellt, mit 900 µl Waschpuffer/Sarkosyl und 3 µl monoklonalen PrP-Antikörper 3F4 über Nacht bei 4°C im Überkopfschüttler inkubiert. Danach erfolgte die Zugabe von Protein-A-Sepharose (150 mg/ml) und eine erneute Inkubation für 90 min im Überkopfschüttler im Kühlraum. Anschließend wurden die Ansätze 5 mal mit 300 µl Waschpuffer/SDS behandelt, wobei die Sepharose nach jedem Waschschritt für 2 min bei 14.000 rpm abzentrifugiert wurde. Nach der letzten Zentrifugation wurde das Pellet in 20 µl 0,1M EtSH/SDS (w/v) 0,5% resuspendiert und 10 min bei 95°C aufgekocht, der Überstand abgenommen und in 20 µl Lysispuffer aufgenommen. Anschließend erfolgte eine Deglykosilierung der di-und monoglykosilierten PrP-Form durch Zusatz von 5µl N-Glykosidase F (5 Units) über Nacht bei 37°C und eine Analyse der unglykosilierten PrP-Form auf einem SDS-Gel (12,5%). Nach beendeter Elektrophorese wurde das Gel 2 h bei 80°C getrocknet und ein Röntgenfilm 1 Tag, 7 Tage bzw. 4 Wochen exponiert. Der Phosphorimager diente der quantitativen Auswertung des ³⁵S-Signals.

| Waschpuffer: | | 3× SEB-Puffer: |
|---|---|---|
| Triton X-100: | 0,5% | 0,25 ml Tris/HCl, pH6,8 1M |
| Desoxycholat: | 0,5% in PBS | 1ml Glycerin (100%) |
| entweder + | 1% SDS (w/v) | 1ml SDS (20%) |
| oder + | 1% Sarkosyl (w/v) | 0,5ml EtSH ad. 3ml H₂O 0,01% Bromphenolblau |

Die verwendeten ScN₂a-Zellen stammen aus einer murinen Neuroblastomzellinie, die zusätzlich zum endogenen Maus-PrP mit einem chimären Maus-Hamster-Maus (MHM₂) PrP-Konstrukt stabil transfiziert wurden [Scott et al., Protein Sci 1 (1992), 986-97]. Die Hamstersequenz (AS 94-131) enhält ein Epitop für den monoklonalen Prionprotein-Antikörper 3F4, welcher für die selektive Immunpräzipitation von PrP im weiteren Verlauf dieses Experiments genutzt wurde.

Kontrollansätze waren sowohl die unselektierte Pool-RNA als auch ein Ansatz ohne RNA. Um statistisch signifikante Aussagen treffen zu können, wurde das Experiment 6 mal wiederholt. Die Stabilität der 2'Amino-2'Deoxy modifizierten Motiv I-RNA (#7) als auch der unselektierten Pool RNA wurde über 12 Stunden in 10%igem FKS in MEM-Medium untersucht. Dabei konnte auch für die Motiv I RNA keine Degradation innerhalb von 12 Stunden festgestellt werden.

Nach insgesamt 12 Stunden radioaktiver Markierung mit ³⁵S-Methionin/Cystein in Gegenwart der RNA (700 nM) erfolgte die Zelllyse, wobei jeweils die Hälfte der postnukleären Überstände nur ultrazentrifigiert wurde (Spur 1-9). Die andere Hälfte wurde erst einer pK-Behandlung unterzogen und anschließend ultrazentrifugiert (Spur 10-18). Durch den Ultrazentrifugationsschritt in Gegenwart von 1% Sarkosyl wird PrP^{Sc} sedimentiert, während PrP^{C} sich im Überstand des Ultrafiltrates befindet.

Danach wurde das sedimentierte PrP^{Sc} gelöst und unter Verwendung des 3F4 Antikörpers immunpräzipitiert. Während durch Ultrazentrifugation der Proben 1-9 die Menge an *de novo* synthetisiertem und unlöslichem PrP^{Sc} erhalten wurde, war in den Proben 10-18 sowohl Unlöslichkeit als auch pK-Resistenz des *de novo* synthetisierten PrP^{SC} Untersuchungskriterium.

Da maximal nur ca. 5% des neu translatierten PrP^{C} in PrP^{Sc} konvertiert werden [Taraboulos et al., Mol Biol Cell 3 (1992), 851-63], wurden die PrP^{Sc}-Proben nach der Immunpräzipitation und vor der Gelanalyse zur Verstärkung des ³⁵S-Signal deglykosyliert. Dadurch konnte die Gesamtmenge an radioaktiv markiertem PrP^{Sc} quantifiziert werden.

Aus Fig. 11 ist ersichtlich, daß das *de novo* synthetisierte, N-terminal trunkierte, immunpräzipitierte und deglykosylierte PrP^{Sc} mit einer Größe von 19 kDa in allen analysierten Proben vorhanden ist. Da bei der Immunpräzipitation des *de novo* synthetisierten PrP^{Sc} ein PrP-spezifischer Antikörper (3F4) verwendet wurde, der nicht an das endogene murine PrP binden kann, ist das erhaltene radioaktive PrP-Signal nur auf das chimäre Maus-Hamster-Maus-Konstrukt zurückzuführen.

Die Quantifizierung dieser Signale zeigt, daß die absolute Menge an *de novo* synthetisierten, unlöslichen PrP^{Sc} durch den Zusatz von Aptamer #7 (Spur 7-9) im Vergleich zur Pool-RNA (Spur 4-6) bzw. dem Pufferansatz (Spur 1-3) nicht beeinflußt wird. Es konnte jedoch eindeutig gezeigt werden, daß der relative Anteil an pK-resistentem Material bezogen auf den Anteil des nicht pk-behandelten, unlöslichen PrP^{Sc} in Gegenwart von Aptamer #7 (Spur 16-18) im Vergleich zu den Kontrollansätzen reduziert wird.

Um dieses Ergebnis zu bestätigen, wurde ein weiteres Experiment mit jeweils 3 Ansätzen pro Versuchsgruppe durchgeführt. In der statistischen Betrachtung der Ergebnisse aller sechs Versuche zeigt sich, daß der Zusatz von Aptamer #7 im Vergleich zur Pool-RNA (p = 0,001) und dem Kontrollansatz ohne RNA (p = 0,004) zu einer signifikanten Reduktion des relativen Anteils an pK-resistenten Material führt.

Die Ansätze ohne RNA bzw. mit Pool-RNA zeigen im Gegensatz dazu bzgl. des relativen Anteils an pK-resistenten Material untereinander keine statistisch signifikanten Unterschiede (p-Wert Pool gegen Puffer= 0,9). In Fig. 12 sind die Ergebnisse der drei Experimentreihen zusammengefaßt und die erhaltenen Durchschnittswerte gegeneinander aufgetragen. Dabei ist zu erkennen, daß die Reduktion des relativen Anteils an *de novo* synthetisierten, pK-resistenten PrP in Gegenwart der Aptamer-RNA # 7 mit 53% im Vergleich zur Pool-RNA mit 22% bzw. der PBS-Kontrolle mit 23% deutlich effektiver ist.

Daraus ergibt sich, dass die erfindungsgemäßen Nukleinsäuren in der Lage sind, den Anteil an pK-resistentem Material, insbesondere Prionprotein, zu verringern.

Die in der vorstehenden Beschreibung, den Ansprüchen, den Zeichnungen und dem Sequenzprotokoll offenbarten Merkmale der Erfindung können sowohl einzeln als auch in beliebiger Kombination für die Verwirklichung der Erfindung in ihren verschiedenen Ausführungsformen wesentlich sein.

## Patentansprüche

1. Nukleinsäure, die an ein Protein umfassend die Aminosäuren 90 bis 129 des Prionproteins bindet.

2. Nukleinsäure, die an ein Peptid umfassend die Aminosäuren 90 bis 129 des Prionproteins bindet.

3. Nukleinsäure, bevorzugterweise eine Nukleinsäure nach Anspruch 1 oder 2, die die Konversion von PrP^{C} zu PrP^{Sc} und/oder die Aggregation von PrP^{Sc} beeinflusst, insbesondere inhibiert.

4. Nukleinsäure, bevorzugterweise eine Nukleinsäure nach einem der Ansprüche 1 bis 3, die die Akkumulation von PrP^{Sc}-Molekülen beeinflusst, insbesondere inhibiert.

5. Nukleinsäure, bevorzugterweise eine Nukleinsäure nach einem der Ansprüche 1 bis 4, die die Übertragbarkeit von Prionenerkrankungen, die Prävention von Prionenerkrankungen, die Amyloidausbildung und Amyloidakkumulation beeinflusst, insbesondere inhibiert.

6. Nukleinsäure, bevorzugterweise eine Nukleinsäure nach einem der Ansprüche 1 bis 5, die die Vermehrung und/oder die Replikation des Erregers und/oder des infektiösen Prionproteins von Erkrankungen beeinflusst, insbesondere inhibiert, wobei die Erkrankung eine neurodegenerative Erkrankung ist und insbesondere die Erkrankung ausgewählt ist aus der Gruppe, die Prionenerkrankungen, spongioforme Encephalopathien und übertragbare spongioforme Encephalopathien umfasst.

7. Nukleinsäure nach einem der Ansprüche 1 bis 6, **dadurch gekennzeichnet, dass** die Nukleinsäure eine Sequenz umfasst, die ausgewählt ist aus der Gruppe, die SEQ ID NO:1, SEQ ID NO:2 und SEQ ID NO:3 umfasst.

8. Nukleinsäure nach einem der Ansprüche 1 bis 7, **dadurch gekennzeichnet, dass** die Nukleinsäure eine Sequenz umfasst, die ausgewählt ist aus der Gruppe, die SEQ ID NO:4 bis SEQ ID NO:18 umfasst.

9. Nukleinsäure nach einem der Ansprüche 1 bis 8, **dadurch gekennzeichnet, dass** die Nukleinsäure eine solche ist, die ausgewählt ist aus der Gruppe, die DNA, RNA, Polynukleotide, Oligonukleotide, Aptamere, Aptazyme, Spiegelmere und Intramere umfasst.

10. Nukleinsäure nach einem der Ansprüche 1 bis 9, **dadurch gekennzeichnet, dass** die Nukleinsäure stabilisiert ist, bevorzugterweise, dass mindestens ein Cytidinrest durch ein 2'-Amino-2'-deoxycytidin und/oder mindestens ein Thymidinrest durch ein 2'-Amino-2'-deoxyuridin ersetzt ist.

11. Vektor, bevorzugterweise ein Expressionsvektor, umfassend eine Nukleinsäure nach einem der Ansprüche 1 bis 10.

12. Zelle umfassend eine Nukleinsäuren nach einem der Ansprüche 1 bis 10 und/oder einen Vektor nach Anspruch 11.

13. Zelle nach Anspruch 12, **dadurch gekennzeichnet, dass** die Zelle eine eukaryontische Zelle, bevorzugterweise eine tierische Zelle und ganz bevorzugterweise eine Zelle eines Säugetiers ist.

14. Zelle nach einem der Ansprüche 12 oder 13, **dadurch gekennzeichnet, dass** die Zelle ausgewählt ist aus der Gruppe, die Saccharomyces cerevisiae und C. elegans umfasst.

15. Tier, bevorzugterweise ein transgenes Tier, umfassend mindestens eine Zelle nach einem der Ansprüche 12 bis 14.

16. Verwendung einer Nukleinsäure nach einem der Ansprüche 1 bis 10 zur Herstellung eines Medikaments, insbesondere zur Behandlung neurodegenerativer Erkrankungen.

17. Verwendung nach Anspruch 16, **dadurch gekennzeichnet, dass** das Medikament ausgewählt ist aus der Gruppe, die Prionenerkrankungen, spongioforme Encephalopathien und übertragbare spongioforme Encephalopathien umfasst.

18. Verwendung nach Anspruch 16, **dadurch gekennzeichnet, dass** das Medikament die Konversion von PrP^{C} zu PrP^{Sc}, die Akkumulation von PrP^{Sc}-Molekülen, die Aggregation von PrP^{Sc} die Amyloidausbildung, die Übertragbarkeit von Prionerkrankungen, die Amyloidakkumulation und/oder die Vermehrung, insbesondere die Replikation des Erregers und/oder des infektiösen Prionproteins für eine Erkrankung beeinflusst, insbesondere inhibiert, wobei die Erkrankung eine neurodegenerative Erkrankung ist und insbesondere die Erkrankung ausgewählt ist aus der Gruppe, die Prionenerkrankungen, spongioforme Encephalopathien und übertragbare spongioforme Encephalopathien umfasst.

19. Verwendung eines Vektors nach Anspruch 11 in der Gentherapie.

20. Verwendung einer Nukleinsäure nach einem der Ansprüche 1 bis 10 zum Nachweis eines Prionproteins.

21. Verwendung einer Nukleinsäure nach einem der Ansprüche 1 bis 10 zur Komplexbildung mit einem Prionprotein.

22. Verwendung nach Anspruch 20 oder 21, **dadurch gekennzeichnet, dass** das Prionprotein ausgewählt ist aus der Gruppe, die PrP^{C}, PrP^{Sc}, PrP^{res}, Prionprotein mit unterschiedlichen Punktmutationen, insbesondere krankheitsrelevante Punktmutationen, Prionprotein mit mehreren Oktapeptidsequenzen, bevorzugterweise mit 4 bis 9 Oktapeptidsequenzen, umfasst.

23. Zusammensetzung, insbesondere pharmazeutische Zusammensetzung, umfassend eine Nukleinsäure nach einem der Ansprüche 1 bis 10, einen Vektor nach Anspruch 11 und/oder eine Zelle nach einem der Ansprüche 12 bis 14 zusammen mit einem geeigneten Trägermaterial.

24. Verfahren zum Screenen von Kandidaten-Verbindungen, die die Wechselwirkung zwischen einem Prionprotein und einer Nukleinsäure nach einem der Ansprüche 1 bis 10 beeinflusst, wobei bevorzugterweise das Prionprotein ausgewählt ist aus der Gruppe, die PrP^{C}, PrP^{Sc}, PrP^{res}, Prionprotein mit unterschiedlichen Punktmutationen, Prionproteine mit mehreren Oktapeptidsequenzen, bevorzugterweise mit 4 bis 9 Oktapeptidsequenzen, umfasst, das die folgenden Schritte umfasst:
a) Bereitstellen des Prionproteins und der Nukleinsäure;
b) optional Bestimmen, ob ein Ereignis eintritt, das ausgewählt ist aus der Gruppe, die die Konversion von PrP^{C} zu PrP^{Sc}, die Akkumulation von PrP^{Sc}-Molekülen, die Aggregation von PrP^{Sc} , die Amyloidbildung, die Übertragbarkeit von Prionerkrankungen, die Prävention von Prionerkrankungen, die Amyloidakkumulation und/oder die Vermehrung, insbesondere die Replikation des Erregers und/oder des infektiösen Prionproteins für eine Erkrankung beeinflusst, insbesondere inhibiert, umfasst, wobei die Erkrankung ausgewählt ist aus der Gruppe, die Prionenerkrankungen, spongioforme Encephalopathien und übertragbare spongioforme Encephalopathien neurodegenerative Erkrankungen umfasst;
c) Hinzufügen der Kandidatenverbindung; und
d) Bestimmen, ob eine Wechselwirkung zwischen der Nukleinsäure und dem Prionprotein beeinflusst wird und/oder es zu einer Konversion von PrP^{C} zu PrP^{Sc}, zu einer Akkumulation von PrP^{Sc}-Molekülen, zu einer Amyloidakkumulation und/oder einer Vermehrung, insbesondere einer Replikation des Erregers für eine Erkrankung kommt und/oder dies inhibiert wird, wobei die Erkrankung ausgewählt ist aus der Gruppe, die Prionenerkrankungen, spongioforme Encephalopathien und übertragbare spongioforme Encephalopathien umfasst.

25. Verwendung des Verfahrens nach Anspruch 24 zur Herstellung eines Medikamentes und/oder zur Ermittlung einer pharmazeutisch wirksamen Verbindung.

26. Verwendung der in dem Verfahren nach Anspruch 24 ermittelten Verbindung zur Herstellung eines Medikaments, wobei die Verbindung die Wechselwirkung zwischen Prionprotein und Nukleinsäure, die Konversion von PrP^{C} zu PrP^{Sc}, die Akkumulation von PrP^{Sc}-Molekülen, die Aggregation von PrP^{Sc}, die Amyloidakkumulation, die Vermehrung, insbesondere die Replikation, und/oder die Transmission des Erregers bzw. des infektiösen Prionproteins für eine Erkrankung beeinflusst, wobei die Erkrankung eine neurodegenerative Erkrankung ist und insbesondere die Erkrankung ausgewählt ist aus der Gruppe, die Prionenerkrankungen, spongioforme Encephalopathien, neurodegenerative Erkrankungen und übertragbare spongioforme Encephalopathien umfasst.

27. Verwendung einer Nukleinsäure nach einem der Ansprüche 1 bis 10 in einem in vitro-Selektionsverfahren.

28. Verfahren zur Identifizierung und Isolierung von Nukleinsäuren, die in der Lage sind, an ein Zielmolekül zu binden, **gekennzeichnet durch** die folgenden Schritte:
- Inkubieren des Zielmoleküls oder eines Teils davon mit einer Vielzahl von Nukleinsäuren, bevorzugterweise einer Nukleinsäurebibliothek, wobei die Nukleinsäuren verschiedene Sequenzen aufweisen;
- Selektieren und Isolieren jener Nukleinsäuren, die in der Lage sind, an das Zielmolekül oder einen Teil davon zu binden;
- optional Amplifizieren der isolierten Nukleinsäure und Wiederholen der ersten beiden Schritte;
- optional Bestimmen der Sequenz und/oder der Bindungsspezifität der isolierten Nukleinsäure,
**dadurch** gekennzeichnet, dass das Zielmolekül ein Protein oder ein Peptid umfassend eine Aminosäuresequenz entsprechend den Aminosäuren 90 bis 129 des Prionproteins ist.

29. Verwendung einer Nukleinsäure nach einem der Ansprüche 1 bis 10 als diagnostisches Mittel.

30. Verwendung einer Nukleinsäure nach einem der Ansprüche 1 bis 10, bevorzugterweise in einem Komplex mit einem Prionprotein, zum rationalen Design von Verbindungen.


SEQUENCE LISTING

<110>  NascaCell GmbH

<120>  Prion-spezifische Aptamere

<130>  N 19004 EP

<160>  18

<170>  PatentIn version 3.1

<210>  1
<211>  34
<212>  DNA
<213>  Künstliche Sequenz

<220>
<221>  misc_feature
<223>  Prion-bindende Nukleinsäuresequenz

<220>
<221>  misc_feature
<222>  (1)..(1)
<223>  K steht für G oder T

<220>
<221>  misc_feature
<222>  (2)..(2)
<223>  K steht für G oder T

<220>
<221>  misc_feature
<222>  (4)..(4)
<223>  M steht für A oder C

<220>
<221>  misc_feature
<222>  (5)..(5)
<223>  Position 5 entweder T oder unbesetzt

<220>
<221>  misc_feature
<222>  (6)..(6)
<223>  R steht für G oder A

<220>
<221>  misc_feature
<222>  (11)..(11)
<223>  R steht für G oder A oder unbesetzt

<220>
<221>  misc_feature
<222>  (13)..(13)
<223>  W steht für A oder T

<220>
<221> misc_feature
<222> (15)..(15)
<223> D steht für A oder G oder T

<220>
<221> misc_feature
<222> (16)..(16)
<223> W steht für  A oder T

<220>
<221> misc_feature
<222> (17)..(17)
<223> K steht für G oder T

<220>
<221> misc_feature
<222> (18)..(18)
<223> R steht für A oder G

<220>
<221> misc_feature
<222> (21)..(21)
<223> K steht für G oder T

<220>
<221> misc_feature
<222> (22)..(22)
<223> Y steht für T oder C oder unbesetzt

<220>
<221> misc_feature
<222> (24)..(24)
<223> W steht für A oder T

<220>
<221> misc_feature
<222> (25)..(25)
<223> M steht für A oder C

<220>
<221> misc_feature
<222> (26)..(26)
<223> K steht für G oder T

<220>
<221> misc_feature
<222> (28)..(28)
<223> K steht für G oder T oder unbesetzt

<220>
<221> misc_feature

<222>  (31)..(31)
<223>  Y steht für T oder C


<220>
<221>  misc_feature
<222>  (34)..(34)
<223>  S steht für G oder C


<400>  1
kkgmtrtgga rgwgdwkrag kygwmkgkgt yggs                                    34


<210>  2
<211>  32
<212>  DNA
<213>  Künstliche Sequenz

<220>
<221>  misc_feature
<223>  Prion-bindende Nukleinsäuresequenz


<220>
<221>  misc_feature
<222>  (1)..(1)
<223>  K steht für G oder T


<220>
<221>  misc_feature
<222>  (2)..(2)
<223>  W steht für A oder T


<220>
<221>  misc_feature
<222>  (4)..(4)
<223>  K steht für G oder T


<220>
<221>  misc_feature
<222>  (5)..(5)
<223>  R steht für A oder G


<220>
<221>  misc_feature
<222>  (6)..(6)
<223>  R steht für A oder G


<220>
<221>  misc_feature
<222>  (7)..(7)
<223>  R steht für A oder G


<220>
<221>  misc_feature
<222>  (8)..(8)

<223>  K steht für G oder T

<220>
<221>  misc_feature
<222>  (12)..(12)
<223>  R steht für A oder G

<220>
<221>  misc_feature
<222>  (13)..(13)
<223>  R steht für A oder G

<220>
<221>  misc_feature
<222>  (15)..(15)
<223>  K steht für G oder T

<220>
<221>  misc_feature
<222>  (16)..(16)
<223>  W steht für A oder T

<220>
<221>  misc_feature
<222>  (19)..(19)
<223>  R steht für A oder G

<220>
<221>  misc_feature
<222>  (20)..(20)
<223>  K steht für G oder T

<220>
<221>  misc_feature
<222>  (21)..(21)
<223>  W steht für A oder T

<220>
<221>  misc_feature
<222>  (22)..(22)
<223>  R steht für A oder G

<220>
<221>  misc_feature
<222>  (24)..(24)
<223>  R steht für A oder G

<220>
<221>  misc_feature
<222>  (26)..(26)
<223>  K steht für G oder T

<220>
<221> misc_feature
<222> (27)..(27)
<223> W steht für A oder T

<220>
<221> misc_feature
<222> (28)..(28)
<223> K steht für G oder T

<220>
<221> misc_feature
<222> (29)..(29)
<223> R steht für A oder G

<220>
<221> misc_feature
<222> (31)..(31)
<223> M steht für A oder C

<220>
<221> misc_feature
<222> (32)..(32)
<223> S steht für G oder C

<400> 2
kwgkrrrkgt grrgkwggrk wrgrakwkra ms 32

<210> 3
<211> 42
<212> DNA
<213> Künstliche Sequenz

<220>
<221> misc_feature
<223> Prion-bindende Nukleinsäuresequenz

<220>
<221> misc_feature
<222> (4)..(4)
<223> Position 4 C C oder unbesetzt

<220>
<221> misc_feature
<222> (37)..(37)
<223> Position 37 G C oder unbesetzt

<220>
<221> misc_feature
<222> (38)..(38)
<223> Position 37 G C oder unbesetzt

<400> 3

tcccgaaggt ggggcgtaga ggggagatgc ggtgcggcct ga 42

<210> 4
<211> 39
<212> DNA
<213> Künstliche Sequenz

<220>
<221> misc_feature
<223> Prion-bindende Nukleinsäuresequenz

<400> 4
gtgctatgga gtggaggagt tgaaggtgtc ggggttggc 39

<210> 5
<211> 39
<212> DNA
<213> Künstliche Sequenz

<220>
<221> misc_feature
<223> Prion-bindende Nukleinsäuresequenz

<400> 5
gtgctatgga gtggaggaga tgaaggtgtc ggggttggc 39

<210> 6
<211> 38
<212> DNA
<213> Künstliche Sequenz

<220>
<221> misc_feature
<223> Prion-bindende Nukleinsäuresequenz

<400> 6
gtgctatgga gtggatgagt gaaggtgtcg gggttggc 38

<210> 7
<211> 39
<212> DNA
<213> Künstliche Sequenz

<220>
<221> misc_feature
<223> Prion-bindende Nukleinsäuresequenz

<400> 7
gtgctatgga gtggaggagt cgaaggtgtc ggggttggc 39

<210> 8
<211> 38
<212> DNA
<213> Künstliche Sequenz

<220>
<221>  misc_feature
<223>  Prion-bindende Nukleinsäuresequenz

<400>  8
gtgctatgga gtgtaggagt cgaagggtcg gggttggc                              38

<210>  9
<211>  39
<212>  DNA
<213>  Künstliche Sequenz

<220>
<221>  misc_feature
<223>  Prion-bindende Nukleinsäuresequenz

<400>  9
gtgctatgga gtgtaggagt tgaaggtgtc ggggttggc                             39

<210>  10
<211>  40
<212>  DNA
<213>  Künstliche Sequenz

<220>
<221>  misc_feature
<223>  Prion-bindende Nukleinsäuresequenz

<400>  10
gtgctatgga agtggaggag ttgaaggtgt cggggttggc                            40

<210>  11
<211>  38
<212>  DNA
<213>  Künstliche Sequenz

<220>
<221>  misc_feature
<223>  Prion-bindende Nukleinsäuresequenz

<400>  11
gtgctatgga gtggaggagt tgaatggtcg gggttggc                              38

<210>  12
<211>  38
<212>  DNA
<213>  Künstliche Sequenz

<220>
<221>  misc_feature
<223>  Prion-bindende Nukleinsäuresequenz

<400>  12

taggaggtgt gaaggtgggg taggatagga agttaacc 38

<210> 13
<211> 37
<212> DNA
<213> Künstliche Sequenz

<220>
<221> misc_feature
<223> Prion-bindende Nukleinsäuresequenz

<400> 13
taggaggtgt gaaggtgggg taggatagga agttacc 37

<210> 14
<211> 37
<212> DNA
<213> Künstliche Sequenz

<220>
<221> misc_feature
<223> Prion-bindende Nukleinsäuresequenz

<400> 14
taggagggtg aaggtggggt aggataggaa gttaacc 37

<210> 15
<211> 40
<212> DNA
<213> Künstliche Sequenz

<220>
<221> misc_feature
<223> Prion-bindende Nukleinsäuresequenz

<400> 15
tcccgaaggt ggggcgtaga ggggagatgc ggtgcgctga 40

<210> 16
<211> 42
<212> DNA
<213> Künstliche Sequenz

<220>
<221> misc_feature
<223> Prion-bindende Nukleinsäuresequenz

<400> 16
tcccgaaggt ggggcgtaga ggggagatgc ggtgcggcct ga 42

<210> 17
<211> 39
<212> DNA
<213> Künstliche Sequenz

<220>
<221>  misc_feature
<223>  Prion-bindende Nukleinsäuresequenz

<400>  17
tccgaaggtg gggcgtagag gggagatgcg gtgcgctga                              39

<210>  18
<211>  40
<212>  DNA
<213>  Künstliche Sequenz

<220>
<221>  misc_feature
<223>  Prion-bindende Nukleinsäuresequenz

<400>  18
atagagtccg gaaagggtca gcggaggctg ggtgtggagg                             40